# EUROPÄISCHE PATENTSCHRIFT

(11) **EP 3 380 448 B1**
(45) Veröffentlichungstag und Bekanntmachung des Hinweises auf die Patenterteilung: **28.08.2019**
(21) Anmeldenummer: 16794962.7
(22) Anmeldetag: 01.11.2016
(51) Int. Cl.: C07C 29/76, C07C 29/10, C07C 29/48

(54) **VERFAHREN ZUR HERSTELLUNG VON 1,2-PROPANDIOL AUS PROPEN UND WASSERSTOFFPEROXID**
METHOD FOR THE PRODUCTION OF 1,2-PROPANE DIOL FROM PROPENE AND HYDROGEN PEROXIDE
PROCÉDÉ DE PRÉPARATION DE 1,2-PROPANEDIOL À PARTIR DE PROPÈNE ET DE PEROXYDE D'HYDROGÈNE

(30) Priorität: 25.11.2015 EP 15196268
(43) Veröffentlichungstag der Anmeldung: 03.10.2018
(73) Patentinhaber: Evonik Degussa GmbH, 45128 Essen (DE)
(72) Erfinder: WIEDERHOLD, Holger, 64297 Darmstadt (DE); BOLZ, David, 60594 Frankfurt (DE); JAEGER, Bernd, 64404 Bickenbach (DE); KÖHLE, Hans-Jürgen, 63533 Mainhausen (DE); IMM, Sebastian, 61118 Bad Vilbel (DE); THIELE, Georg Friedrich, 61169 Friedberg (DE)
(74) Vertreter: Evonik Patent Association
(86) Internationale Anmeldenummer: PCT/EP2016/076270
(87) Internationale Veröffentlichungsnummer: WO 2017/089075

(56) Entgegenhaltungen:
- US-A- 4 308 409
- C. VENTURELLO ET.AL.: "A new, effective catalytic system for epoxidation of olefins by hydrogen peroxide under phase-transfer conditions", J. ORG. CHEM., Bd. 48, 1983, Seiten 3831-3833, XP002756846,
- JIAN LI ET.AL.: "Influence of composition of heteropolyphosphatotungstate catalyst on epoxidation of propylene", JOURNAL OF MOLECULAR CATALYSIS A: CHEMICAL, Bd. 218, Nr. 2, 24. August 2004 (2004-08-24), Seiten 247-252, XP002756847, DOI: 10.1016/j.molcata.2004.04.024

## Beschreibung

Die Erfindung betrifft ein Verfahren zur Herstellung von 1,2-Propandiol aus Propen und Wasserstoffperoxid, das keine Isolierung und Reinigung von Propenoxid erfordert.

1,2-Propandiol wird technisch durch Umsetzung von Propenoxid mit Wasser hergestellt. Propenoxid wird technisch durch Epoxidierung von Propen hergestellt. Bei dem etablierten Verfahren wird Propenoxid aus der Reaktionsmischung der Epoxidierung isoliert und gereinigt, bevor es zu 1,2-Propandiol umgesetzt wird.

Bei der Herstellung von Propenoxid nach dem HPPO-Verfahren, bei dem Propen und Wasserstoffperoxid in Gegenwart eines Titansilicalits in Methanol als Lösungsmittel umgesetzt werden, werden als Nebenprodukte 1,2-Propandiol und 1,2-Propandiolmonomethylether erhalten. In WO 04/009568 wird vorgeschlagen, aus der Reaktionsmischung des HPPO-Verfahrens durch Destillation ein Roh-Propenoxid mit einem Gehalt an 95 bis 99 % herzustellen, dieses ohne weitere Reinigung mit Wasser zu 1,2-Propandiol umzusetzen und zusammen mit aus dem Sumpfprodukt der Destillation abgetrennten Nebenprodukt 1,2-Propandiol zu reinigen. Auch bei diesem Verfahren wird Propenoxid isoliert und in einem separaten Reaktor zu 1,2-Propandiol umgesetzt.

J. Guojie et al. beschreiben in Chinese Journal of Catalysis 26 (2005) 1005-1010 die Epoxidierung von Propen mit Wasserstoffperoxid in Gegenwart eines quaternären Ammoniumheterpolyphosphatowolframats als Katalysator in einer zweiphasigen Reaktionsmischung mit CHCl₃ oder einer Mischung von Toluol und Tributylphosphat als Lösungsmittel. Zur Verbesserung der Selektivität von Propenoxid werden K₂HPO₄ oder Na₂HPO₄ als Additiv zugesetzt. Ohne Zusatz an Additiv wird mehr 1,2-Propandiol als Propenoxid erhalten mit Selektivitäten für 1,2-Propandiol von 41,2 und 56,3 %.

J. Kaur et al. beschreiben in Catal. Commun. 5 (2004) 709-713 die Epoxidierung von Propen mit Wasserstoffperoxid in Gegenwart von Methyltrioctylammoniumperoxopolywolframat. Die Epoxidierung wird entweder in einer durch Zusatz des Tensids Brij® 30 hergestellten Mikroemulsion oder in einem Zweiphasensystem mit 1,2-Dichlorethan als Lösungsmittel durchgeführt. Für das Zweiphasensystem werden zur Wiederverwendung des Katalystors die Phasen getrennt, die organische Phase mit Wasser extrahiert und Propenoxid und nicht umgesetztes Propen durch Spülen mit Stickstoff bei 60 °C entfernt. Für die Mikroemulsion wird zur Wiederverwendung des Katalystors eine Membranultrafiltration zur Abtrennung von Katalysator und Wasser vorgeschlagen.

S. R. Chowdhury et al., Chem. Eur. J. 12 (2006) 3061-3066 beschreiben eine Epoxidierung von Cycloocten mit Wasserstoffperoxid in Gegenwart von [CH₃N(C₈H₁₇)₃]₁₂[WZn₃(ZnW₉O₃₄)₂] als Katalysator und Toluol als Lösungsmittel mit Abtrennung des Katalysators durch Filtration über Aluminiumoxid-Membranen mit mittleren Porenradien von 2,3 nm und 4,3 nm. Mit der Aluminiumoxid-Membran mit einem mittleren Porenradius von 2,3 nm wurde auch Na₁₂[WZn₃(ZnW₉O₃₄)₂] aus einer wässrigen Lösung abgetrennt.

S. S. Luthra et al., J. Membr. Sci. 201 (2002) 65-75 beschreiben die Abtrennung der Phasentransferkatalysatoren Tetra-n-butylammoniumbromid und Tetra-n-octylammoniumbromid aus Toluol durch Nanofiltration.

US 4 308 409 offenbart ebenfalls ein Verfahren zur Herstellung von 1,2-Propandiol aus Propen.

Es wurde nun gefunden, dass sich Propen mit Wasserstoffperoxid in hohen Ausbeuten und Selektivitäten in einer Stufe zu 1,2-Propandiol umsetzen lassen, wenn die Reaktion mit einer Kombination aus einem Phasentransferkatalysator und einem Heteropolywolframat in einer Reaktionsmischung mit zwei Flüssigphasen durchgeführt wird, der pH der wässrigen Phase unter 6 gehalten wird, das in der organischen Phase der Reaktionsmischung enthaltene Propenoxid in den Reaktor zurückgeführt wird und 1,2-Propandiol aus der wässrigen Phase abgetrennt wird. Gegenstand der Erfindung ist demnach ein Verfahren zur Herstellung von 1,2-Propandiol aus Propen und Wasserstoffperoxid umfassend die Schritte
a) Umsetzen von Propen mit Wasserstoffperoxid in Gegenwart einer Katalysatormischung umfassend einen Phasentransferkatalysator und ein Heteropolywolframat, wobei die Umsetzung in einer flüssigen Mischung umfassend eine wässrige Phase mit einem pH von maximal 6 und eine organische Phase durchgeführt wird,
b) Trennen der zweiphasigen Mischung von Schritt a) in eine wässrige Phase P1 und eine organische Phase P2,
c) Rückführen des in der abgetrennten organischen Phase P2 enthaltenen Propenoxids in die Umsetzung von Schritt a) und
d) Abtrennen von 1,2-Propandiol aus der in Schritt b) abgetrennten wässrigen Phase P1.

Bei dem erfindungsgemäßen Verfahren wird in Schritt a) Propen mit Wasserstoffperoxid in Gegenwart einer Katalysatormischung umgesetzt, die einen Phasentransferkatalysator und ein Heteropolywolframat umfasst. Die Umsetzung wird in einer flüssigen Mischung durchgeführt, die zwei flüssige Phasen umfasst, eine wässrige Phase und eine organische Phase.

Propen kann in reiner Form oder in Mischung mit Propan eingesetzt werden, wobei der Anteil an Propan bis 20 mol-% betragen kann. Vorzugsweise beträgt der Anteil an Propan im eingesetzten Propen weniger als 5 mol-%.

Wasserstoffperoxid wird vorzugsweise in Form einer wässrigen Lösung eingesetzt, vorzugsweise mit einem Gehalt an Wasserstoffperoxid von 10 bis 80 Gew.-%, besonders bevorzugt mit einem Gehalt an Wasserstoffperoxid von 30 bis 70 Gew.-%. Im erfindungsgemäßen Verfahren kann das Wasserstoffperoxid-Rohprodukt eingesetzt werden, dass im Anthrachinonverfahren zur Herstellung von Wasserstoffperoxid in der Extraktionsstufe erhalten wird.

Die wässrige Phase enthält Wasser, nicht umgesetztes Wasserstoffperoxid und gebildetes 1,2-Propandiol. Die organische Phase enthält Propen und als Zwischenprodukt gebildetes Propenoxid und kann außerdem aus dem eingesetzten Propen stammendes Propan enthalten. Zusätzlich kann die organische Phase mindestens ein mit Wasser nicht mischbares Lösungsmittel enthalten.

Die im erfindungsgemäßen Verfahren verwendete Katalysatormischung umfasst ein Heteropolywolframat, wobei das Heteroatom vorzugsweise Phosphor oder Arsen ist und besonders bevorzugt Phosphor ist, d.h. besonders bevorzugt ist das Heteropolywolframat ein Polywolframatophosphat. Heteropolywolframate sind dem Fachmann aus dem Stand der Technik bekannt. Am meisten bevorzugt sind Polywolframatophosphate mit einem molaren Verhältnis von Phosphor zu Wolfram im Bereich von 1:2 bis 1:12. Vorzugsweise wird Polywolframatophosphat in der flüssigen Mischung in Schritt a) in situ aus Phosphorsäure und Natriumwolframat erzeugt, wobei Phosphorsäure und Natriumwolframat vorzugsweise mit einem molaren Verhältnis von Phosphor zu Wolfram im Bereich von 1:2 bis 10:1 und besonders bevorzut von 4:1 bis 8:1 eingesetzt werden. Aus einem Polywolframatophosphat bilden sich in der wässrigen Phase mit Wasserstoffperoxid Peroxowolframate und Peroxowolframatophosphate, beispielsweise PO₄[WO(O₂)₂]₄³⁻ und HPO₄MO(O₂)₂]₂²⁻ sowie deren teilweise protonierte Formen.

Die im erfindungsgemäßen Verfahren verwendete Katalysatormischung umfasst außerdem einen Phasentransferkatalysator. Der Phasentransferkatalysator umfasst ein Kation oder eine Verbindung, die in der wässrigen Phase ein Kation bildet, wobei das Kation mit einem Peroxowolframat oder Heteropolyperoxowolframat ein in der organischen Phase lösliches Salz bilden kann. Vorzugsweise umfasst der Phasentransferkatalysator ein einfach geladenes Kation oder eine Verbindung, die in der wässrigen Phase ein einfach geladenes Kation bildet. Als Phasentransferkatalysator eignen sich quaternäre Ammoniumsalze, tertiäre Amine oder quaternäre Phosphoniumsalze. Geeignete quaternäre Ammoniumsalze sind Tetraalkylammoniumsalze mit insgesamt mindestens 12 Kohlenstoffatomen in den Alkylgruppen, beispielsweise Dodecyltrimethylammoniumsalze, Hexadecyltrimethylammoniumsalze, Octadecyltrimethylammoniumsalze, Methyltributylammoniumsalze und Methyltrioctylammoniumsalze. Geeignet sind quaternäre Ammoniumsalze mit ein- oder zweiwertigen Anionen, beispielsweise Chlorid, Bromid, Nitrat, Sulfat, Hydrogenphosphat, Dihydrogenphosphat, Methylsulfonat, Methylsulfat und Ethylsulfat. Geeignete tertiäre Amine sind Dodecyldimethylamin, Hexadecyldimethylamin, Octadecyldimethylamin, Tributylamin und Trioctylamin. Der Phasentransferkatalysator wird vorzugsweise in einer Menge eingesetzt, die in der flüssigen Mischung ein molares Verhältnis von Phasentransferkatalysator zu Wolfram im Bereich von 0,2:1 bis 3:1 und besonders bevorzugt von 0,4:1 bis 1:1 ergibt, wobei sich das molare Verhältnis auf die im eingesetzten Phasentransferkatalysator enthaltenen Kationen oder Kationen bildende Verbindungen und auf die eingesetzte Menge an Wolfram bezieht.

In einer bevorzugten Ausführungsform umfasst der Phasentransferkatalysator mindestens ein Salz mit einem tertiären oder quaternären Ammoniumion der Struktur R¹R²R³R⁴N⁺, worin R¹ ein Rest Y-O(C=O)R⁵ ist, wobei Y für einen der Reste CH₂CH₂, CH(CH₃)CH₂ und CH₂CH(CH₃) steht und R⁵ ein Alkylrest oder Alkenylrest mit 11 bis 21 Kohlenstoffatomen ist, R² Wasserstoff oder ein Alkylrest mit 1 bis 4 Kohlenstoffatomen ist und R³ und R⁴ unabhängig voneinander R¹, ein Alkylrest mit 1 bis 4 Kohlenstoffatomen oder Y-OH sind. Bevorzugt sind quaternäre Ammoniumsalze mit Methylsulfat als Anion, in denen R² ein Methylrest und R⁵ ein linearer Alkylrest oder Alkenylrest ist. Besonders bevorzugt sind die Salze (CH₃)₃N⁺CH₂CH₂O(C=O)R⁵ CH₃OSO₃⁻, (CH₃)₂N⁺(CH₂CH₂OH)(CH₂CH₂O(C=O)R⁵) CH₃OSO₃⁻, (CH₃)₂N⁺(CH₂CH₂O(C=O)R⁵)₂ CH₃OSO₃⁻, CH₃N⁺(CH₂CH₂OH)₂(CH₂CH₂O(C=O)R⁵) CH₃OSO₃⁻, CH₃N⁺(CH₂CH₂OH)(CH₂CH₂O(C=O)R⁵)₂ CH₃OSO₃⁻, CH₃N⁺(CH₂CH₂O(C=O)R⁵)₃ CH₃OSO₃⁻, (CH₃)₃N⁺CH₂CH(CH₃)O(C=O)R⁵ CH₃OSO₃⁻, (CH₃)₂N⁺(CH₂CH(CH₃)OH)(CH₂CH(CH₃)O(C=O)R⁵) CH₃OSO₃⁻ und (CH₃)₂N⁺(CH₂CH(CH₃)O(C=O)R⁵)₂ CH₃OSO₃⁻, in denen jeweils R⁵ ein linearer Alkylrest oder Alkenylrest mit 11 bis 21 Kohlenstoffatomen ist. Am meisten bevorzugt ist das Salz (CH₃)₂N⁺(CH₂CH(CH₃)O(C=O)R⁵)₂ CH₃OSO₃⁻ in dem R⁵ ein Alkylrest oder Alkenylrest mit 11 bis 17 Kohlenstoffatomen ist. Die Phasentransferkatalysatoren dieser Ausführungsform lassen sich durch Verestern von Ethanolamin, Isopropanolamin, Diethanolamin, Diisopropanolamin, Triethanolamin oder Triisopropanolamin mit einer Fettsäure und anschließendes Quaternisieren mit Dimethylsulfat herstellen und haben gegenüber Tetraalkylammoniumsalzen den Vorteil, dass sie gut biologisch abbaubar sind und das im erfindungsgemäßen Verfahren anfallende Abwasser ohne weitere Vorbehandlung einer biologischen Kläranlage zugeführt werden kann. Mit den bevorzugten Salzen mit Methylsulfat als Anion kann außerdem die Korrosivität der Reaktionsmischung im Vergleich zu Tetraalkylammoniumhalogeniden verringert werden. Der Phasentransferkatalysator wird bei dieser Ausführungsform vorzugsweise gemischt mit mindestens einem Lösungsmittel ausgewählt aus Ethanol und 2-Propanol der flüssigen Mischung der Umsetzung zugegeben. Durch die Verwendung dieser Lösungsmittel lässt sich der Phasentransferkatalysator besser dosieren und in der flüssigen Mischung verteilen.

Der Phasentransferkatalysator und das Heteropolywolframat können der Umsetzung als Mischung oder getrennt voneinander zugeführt werden. Vorzugsweise werden Phasentransferkatalysator und Heteropolywolframat im Schritt a) getrennt zugeführt.

Die Umsetzung von Propen mit Wasserstoffperoxid wird bei einem pH der wässrigen Phase von maximal 6 durchgeführt. Vorzugsweise wird der pH der wässrigen Phase im Bereich von 1,0 bis 3,5 gehalten, besonders bevorzugt im Bereich von 2,0 bis 3,0. Der pH kann dabei durch Zugabe von Säure, vorzugsweise Schwefelsäure oder Phosphorsäure, oder durch Zugabe von Base, vorzugsweise Natronlauge, in diesem Bereich gehalten werden. Der Begriff pH bezieht sich dabei auf den mit einer Glaselektrode gemessenen scheinbaren pH Wert, wobei die Glaselektrode mit wässrigen Pufferlösungen kalibriert wird. Durch die Einstellung eines pH im bevorzugten Bereich lässt sich eine hohe Selektivität für 1,2-Propandiol erreichen und eine Anreicherung von Propenoxid in der wässrigen Phase verhindern, was die nachfolgende Abtrennung von 1,2-Propandiol aus der wässrigen Phase vereinfacht.

Die Umsetzung von Propen mit Wasserstoffperoxid wird vorzugsweise mit einem molaren Überschuss an Propen durchgeführt, wobei Propen vorzugsweise in einem molaren Verhältnis von Propen zu Wasserstoffperoxid von 1,1:1 bis 10:1 eingesetzt wird.

Die Umsetzung wird vorzugsweise bei einer Temperatur im Bereich von 30 bis 100 °C, besonders bevorzugt 70 bis 90 °C durchgeführt. Die Umsetzung erfolgt vorzugsweise bei einem Druck, der höher ist als der Sättigungsdampfdruck von Propen bei der Temperatur der Umsetzung, sodass der größte Teil des Propens in der organischen Phase der flüssigen Mischung vorliegt.

Die Umsetzung von Propen mit Wasserstoffperoxid kann mit oder ohne Zusatz von Lösungsmitteln durchgeführt werden. Vorzugsweise wird die Umsetzung in Gegenwart von mindestens einem Lösungsmittel durchgeführt, das einen Siedepunkt von mehr als 100 °C, vorzugsweise mehr als 120 °C, sowie eine Wasserlöslichkeit bei 20 °C von weniger als 250 mg/kg aufweist. Als Lösungsmittel können Alkohole mit einer oder mehreren Hydroxygruppen, Ether, Ester, Ketone oder alkylierte aromatische Kohlenwasserstoffe verwendet werden. Durch die Verwendung eines Lösungsmittels lässt sich der Anteil an Heteropolywolframat in der organischen Phase erhöhen. Vorzugsweise wird der Anteil an Lösungsmittel so gewählt, dass der Anteil des Lösungsmittels an der organischen Phase während der Umsetzung im Bereich von 10 bis 90 Gew.-% liegt.

In einer besonders bevorzugten Ausführungsform umfasst das Lösungsmittel einen epoxidierten Fettsäuremethylester. Dazu kann an Stelle des epoxidierter Fettsäuremethylesters auch der entsprechende Fettsäuremethylester mit ungesättigten Fettsäureresten eingesetzt werden, der in der flüssigen Mischung von Schritt a) zum epoxidierten Fettsäuremethylester umgesetzt wird. Am meisten bevorzugt sind epoxidierte Fettsäuremethylester, deren Fettsäurereste aus Pflanzenölen stammen, insbesondere aus Sojaöl. Die epoxidierten Fettsäuremethylester haben den Vorteil, dass sie in der wässrigen Phase wenig löslich sind und keine Abtrennung von Lösungsmittel aus der wässrigen Phase der Umsetzung erforderlich wird.

In einer weiteren bevorzugten Ausführungsform umfasst das Lösungsmittel einen alkylierten aromatischen Kohlenwasserstoff mit 8 bis 12 Kohlenstoffatomen. Geeignete alkylierte aromatische Kohlenwasserstoffe sind beispielsweise 1,2-Dimethylbenzol (o-Xylol), 1,3-Dimethylbenzol (m-Xylol), 1,4-Dimethylbenzol (p-Xylol), Ethylbenzol, 1,2,3-Trimethylbenzol, 1,2,4-Trimethylbenzol, 1,3,5-Trimethylbenzol (Mesitylen), 1-Ethyl-2-methylbenzol, 1-Ethyl-3-methylbenzol und 1-Ethyl-4-methylbenzol und n-Propylbenzol. Vorzugsweise werden als Lösungsmittel Kohlenwasserstoffmischungen verwendet, die mehr als 50 Gew.-%, besonders bevorzugt mehr als 80 Gew,-%, alkylierte aromatische Kohlenwasserstoffe mit 8 bis 12 Kohlenstoffatomen enthalten. Durch die Verwendung eines Lösungsmittels, das alkylierte aromatische Kohlenwasserstoff mit 8 bis 12 Kohlenstoffatomen umfasst, lässt sich eine weitgehende Extraktion des Heteropolywolframats in die organische Phase der Reaktionsmischung erreichen, sodass eine verbesserte Rückführung des Heteropolywolframats mit der organischen Phase und eine vereinfachte Rückgewinnung von Heteropolywolframat aus der organischen Phase der Umsetzung von Propen mit Wasserstoffperoxid erreicht werden kann.

Der Phasentransferkatalysator, das molare Verhältnis von Phasentransferkatalysator zu Heteropolywolframat, das molare Verhältnis von Heteroatom des Heteropolywolframats zu Wolfram, das molare Verhältnis von Propen zu Wasserstoffperoxid und Art und Menge eines optional eingesetzten Lösungsmittels werden vorzugsweise so gewählt, dass ein möglichst großer Teil des in der flüssigen Mischung vorliegenden Wolframs durch den Phasentransferkatalysator in die organische Phase der flüssigen Mischung transferiert wird. Vorzugsweise wird dazu einer der oben genannten Phasentransferkatalysatoren auf Basis eines Alkanolaminfettsäureesters in Kombination mit einem epoxidierten Fettsäuremethylester oder einer Kohlenwasserstoffmischungen mit mehr als 50 Gew.-% alkylierten aromatischen Kohlenwasserstoffen mit 8 bis 12 Kohlenstoffatomen als Lösungsmittel eingesetzt.

Die Umsetzung von Propen mit Wasserstoffperoxid kann satzweise oder kontinuierlich durchgeführt werden, wobei eine kontinuierliche Umsetzung bevorzugt ist. Bei einer kontinuierlichen Umsetzung liegt die Konzentration an Wasserstoffperoxid in der wässrigen Phase vorzugsweise im Bereich von 0,1 bis 5 Gew.-%, besonders bevorzugt 0,5 bis 3 Gew.-%. Eine solche Konzentration an Wasserstoffperoxid kann durch die Wahl der Reaktionstemperatur, des molaren Verhältnisses von Propen zu Wasserstoffperoxid und der Verweilzeit der flüssigen Mischung im Reaktor, in dem die Umsetzung erfolgt, eingestellt werden.

Während der Umsetzung wird die flüssige Mischung vorzugsweise gemischt, um eine hohe Phasengrenzfläche zwischen der wässrigen Phase und der organischen Phase zu erzeugen. Dazu wird vorzugsweise die Umsetzung kontinuierlich in einem Schlaufenreaktor durchgeführt, der unbewegliche Einbauten aufweist, und die flüssige Mischung wird mit einer Strömungsgeschwindigkeit durch den Schlaufenreaktor geführt, die an den Einbauten eine turbulente Strömung erzeugt. Als Einbauten können dazu Blenden, statische Mischelemente, strukturierte Packungen oder Füllkörperschüttungen eingesetzt werden. Alternativ oder in Kombination dazu können als Einbauten Wärmetauscher, wie Plattenwärmetauscher oder Rohrbündelwärmetauscher eingesetzt werden, bei denen zwischen den Platten oder in den Rohren des Rohrbündels eine turbulente Strömung erzeugt wird.

In Schritt b) des erfindungsgemäßen Verfahrens wird die zweiphasige Mischung von Schritt a) in eine wässrige Phase P1 und eine organische Phase P2 getrennt. Die Trennung wird vorzugsweise in einem Abscheidegefäß durchgeführt, wobei zur Unterstützung der Trennung die zweiphasige Mischung über ein Koaleszierelement geleitet werden kann, das eine Packung oder Schüttung mit einer Oberfläche enthält, die von der in der zweiphasigen Mischung dispers vorliegenden Phase benetzt wird.

Die Trennung der flüssigen Phasen wird in Schritt b) vorzugsweise in Gegenwart einer Gasphase durchgeführt. Bei der Umsetzung in Schritt a) kann es zu einer Zersetzung von Wasserstoffperoxid unter Bildung von Sauerstoff kommen und die Gasphase kann in Schritt b) dann Sauerstoff enthalten. Um die Bildung einer zündfähigen Gasphase zu vermeiden, wird in Schritt b) deshalb vorzugsweise durch Zufuhr von Inertgas und Entnahme eines Gasstroms der Sauerstoffgehalt dieser Gasphase auf weniger als 7 Vol. % gehalten. Als Inertgas können Stickstoff, Argon, Kohlendioxid oder Methan verwendet werden, wobei Stickstoff bevorzugt ist.

In Schritt c) des erfindungsgemäßen Verfahrens wird das in der organischen Phase P2 enthaltene Propenoxid in die Umsetzung von Schritt a) zurückgeführt, um eine möglichst vollständige Umsetzung von Propen zu 1,2-Propandiol zu erzielen. Vorzugsweise wird zusätzlich das in der organischen Phase P2 enthaltene Heteropolywolframat in die Umsetzung von Schritt a) zurückgeführt, wobei besonders bevorzugt der in der organischen Phase enthaltene Teil der Katalysatormischung im Wesentlichen vollständig in Schritt a) zurückgeführt wird. Ebenfalls bevorzugt wird das in der organischen Phase P2 enthaltene Propen in die Umsetzung von Schritt a) zurückgeführt. Wenn Propen als Mischung mit Propan eingesetzt wird, wird bei dem Zurückführen in Schritt a) vorzugsweise aus der organischen Phase P2 die gleiche Menge an Propan abgetrennt, die Schritt a) mit der Mischung von Propen mit Propan zugeführt wird. Bei einer kontinuierlichen Durchführung der Umsetzung in Schritt a) lässt sich so eine Anreicherung von Propan in der organischen Phase in Schritt a) vermeiden.

In einer bevorzugten Ausführungsform des erfindungsgemäßen Verfahrens wird die in Schritt b) abgetrennte organische Phase P2 (9) vollständig oder zum Teil in die Umsetzung von Schritt a) zurückgeführt.

In einer weiteren bevorzugten Ausführungsform des erfindungsgemäßen Verfahrens wird in Schritt c) die organische Phase P2 ganz oder teilweise durch Nanofiltration in ein an Heteropolywolframat angereichertes Retentat und ein an Heteropolywolframat abgereichertes Permeat getrennt und das Retentat in die Umsetzung von Schritt a) zurückgeführt. Vorzugsweise wird die gesamte organische Phase P2 durch Nanofiltration in ein Retentat und ein Permeat getrennt. Der Begriff Nanofiltration bezeichnet dabei entsprechend der Nomenklaturempfehlung der IUPAC eine druckgetriebene Trennung an einer Membran, bei der die Membran Partikel und gelöste Moleküle mit einem Durchmesser von weniger als 2 nm zurückhält. Für die Nanofiltration in Schritt c) wird eine Nanofiltrationsmembran eingesetzt, die das Salz aus Peroxowolframat oder Heteropolyperoxowolframat und dem Kation des Phasentransferkatalysators im Retentat zurückhält und Propen mit dem Permeat passieren lässt. Die Nanofiltration wird dabei vorzugsweise so geführt, dass im Retentat die Konzentration des Salzes aus Peroxowolframat oder Heteropolyperoxowolframat und dem Kation des Phasentransferkatalysators nicht über die Sättigungskonzentration ansteigt. Für die Nanofiltration können Membranen auf Basis der Polymere Polyimid, Polyethersulfon, Polyamid und Polydimethylsiloxan eingesetzt werden. Geeignete Nanofiltrationsmembranen sind kommerziell erhältlich, beispielsweise von Evonik Membrane Extraction Technology MET unter der Bezeichnung PuraMem® S600, von GMT Membrantechnik unter der Bezeichnung ONF-2, von SolSep unter den Bezeichnungen 010306, 030306, 030705 und 030306F, sowie von AMS Technologies unter der Bezeichnung NanoPro™ SX. Vorzugsweise wird eine der aus DE 195 07 584, EP 1 741 481 und WO 2011/067054 bekannten Kompositmembranen eingesetzt.

Die Nanofiltration erfolgt vorzugsweise als Querstromfiltration, vorzugsweise bei einer Temperatur im Bereich von 20 bis 90 °C, besonders bevorzugt 40 bis 80 °C. Der Transmembrandruck beträgt vorzugsweise 2 bis 5 MPa. Der Druck auf der Retentatseite kann bis 10 MPa betragen. Der Druck auf der Permeatseite wird vorzugsweise höher gewählt als der niedrigste Druck in den Schritten a) und b) des Verfahrens um ein Ausgasen gelöster Komponenten auf der Permeatseite zu vermeiden.

In einer ebenfalls bevorzugten Ausführungsform wird die organische Phase P2 durch Nanofiltration in ein an Heteropolywolframat angereichertes Retentat und ein an Heteropolywolframat abgereichertes Permeat getrennt, aus dem Permeat wird durch Destillation ein Strom S1, umfassend nicht umgesetztes Propen und als Zwischenprodukt gebildetes Propenoxid, abgetrennt und dieser Strom S1 wird in die Umsetzung von Schritt a) zurückgeführt. Die Destillation wird vorzugsweise bei einem Druck durchgeführt, bei dem durch Kühlen mit Wasser Propen mit dem Destillat kondensiert werden kann. Alternativ kann die Destillation auch bei einem geringeren Druck durchgeführt werden, mit dem Destillat Propenoxid und nur ein Teil des Propens kondensiert werden und der verbleibende Dampf für eine Kondensation von Propen komprimiert werden. Bei dieser Ausführungsform können mit dem Sumpfprodukt der Destillation hochsiedende Nebenprodukte und Abbauprodukte des Phasentransferkatalysators ausgeschleust werden und bei einer kontinuierlichen Durchführung der Umsetzung in Schritt a) eine Anreicherung von schlecht wasserlöslichen Nebenprodukten und Verunreinigungen in der organischen Phase in Schritt a) vermeiden werden. Durch eine destillative Abtrennung von Strom S1 nach der Nanofiltration lässt sich verhindern, dass durch ein Erhitzen eine weitere Reaktion von als Zwischenprodukt gebildetem Propenoxid mit dem Katalysatorsystem erfolgt, die zu Nebenprodukten führt. Wenn in Schritt a) des Verfahrens die Umsetzung in Gegenwart eines Lösungsmittels erfolgt, wird vorzugsweise im Anschluss an die Destillation zur Abtrennung von Strom S1 das Sumpfprodukt dieser Destillation einer weiteren Destillation zugeführt, in der Lösungsmittel durch Destillation abgetrennt wird. Das abgetrennte Lösungsmittel kann in Schritt a) zurückgeführt werden. Wenn Propen als Mischung mit Propan eingesetzt wird, wird die Destillation vorzugsweise so durchgeführt, dass zusätzlich zu Strom S1 ein weiterer Strom erhalten wird, der im Wesentlichen aus Propen und Propan besteht und aus dem Propenoxid abgetrennt ist. Aus diesem weiteren Strom wird Propan ganz oder teilweise abgetrennt und das dabei erhaltene, von Propan getrennte oder an Propan abgereicherte Propen wird vorzugsweise in Schritt a) zurückgeführt. Vorzugsweise wird dabei die gleiche Menge an Propan abgetrennt, die Schritt a) mit der Mischung von Propen mit Propan zugeführt wird. Die Destillation des Permeats kann dazu in zwei Stufen durchgeführt werden, wobei in der ersten Destillationsstufe der im Wesentlichen aus Propen und Propan bestehende weitere Strom abgetrennt wird und anschließend in der zweiten Destillationsstufe Strom S1 abgetrennt wird. Vorzugsweise wird die Destillation aber in nur einer Kolonne mit Seitenabzug durchgeführt, Strom S1 als Seitenabzug entnommen und der im Wesentlichen aus Propen und Propan bestehende weitere Strom als Kopfprodukt der Kolonne entnommen.

In einer weiteren bevorzugten Ausführungsform wird die organische Phase P2 durch Destillation in einen Strom S1, umfassend nicht umgesetztes Propen und als Zwischenprodukt gebildetes Propenoxid, sowie einen an Propen und Propenoxid abgereicherten Strom S2 aufgetrennt, der Strom S2 durch Nanofiltration in ein an Heteropolywolframat angereicherte Retentat und ein an Heteropolywolframat abgereicherte Permeat getrennt und der Strom S1 in die Umsetzung von Schritt a) zurückgeführt. Diese Ausführungsform wird vorzugsweise eingesetzt, wenn in Schritt a) des Verfahrens die Umsetzung in Gegenwart eines Lösungsmittels erfolgt und die Destillation wird dann so durchgeführt, dass das Lösungsmittel in Strom S2 bleibt. Das Lösungsmittel kann dann aus dem Permeat der Nanofiltration abgetrennt werden, vorzugsweise durch Destillation, und in die Umsetzung von Schritt a) zurückgeführt werden. Gegenüber der zuvor beschriebenen Ausführungsform hat die Ausführungsform mit einer Abtrennung von Strom S1 vor der Nanofiltration den Vorteil, dass ein wesentlich kleinerer Strom durch die Nanofiltration aufgetrennt wird, was die Apparategröße und den Energieverbrauch der Nanofiltration verringert. Wenn Propen als Mischung mit Propan eingesetzt wird, wird bei dieser Ausführungsform vorzugsweise aus dem Strom S1 durch eine zusätzliche Destillation ein weiterer Strom abgetrennt, der im Wesentlichen aus Propen und Propan besteht und aus dem Propenoxid abgetrennt ist, bevor Strom S1 in Schritt a) zurückgeführt wird. Aus diesem weiteren Strom wird Propan ganz oder teilweise abgetrennt und das dabei erhaltene, von Propan getrennte oder an Propan abgereicherte Propen wird vorzugsweise in Schritt a) zurückgeführt. Vorzugsweise wird dabei die gleiche Menge an Propan abgetrennt, die Schritt a) mit der Mischung von Propen mit Propan zugeführt wird.

In Schritt d) des erfindungsgemäßen Verfahrens wird 1,2-Propandiol aus der in Schritt b) abgetrennten wässrigen Phase P1 abgetrennt. Die Abtrennung von 1,2-Propandiol aus der wässrigen Phase kann durch Destillation erfolgen, vorzugsweise durch eine zweistufige Destillation, bei der in der ersten Stufe Wasser abdestilliert wird und in der zweiten Stufe aus dem Sumpfprodukt der ersten Stufe 1,2-Propandiol abdestilliert wird.

Vorzugsweise werden vor der Abtrennung von 1,2-Propandiol Peroxide durch eine katalytische Hydrierung entfernt. Die Hydrierung erfolgt vorzugsweise mit einem geträgerten Hydrierkatalysator, der ein oder mehrere Metalle aus der Gruppe Ru, Rh, Pd, Pt, Ag, Ir, Fe, Cu, Ni und Co auf einem Träger enthält, wobei als Träger Aktivkohle, SiO₂, TiO₂, ZrO₂, Al₂O₃ und Aluminiumsilikate bevorzugt sind. Bevorzugt sind Hydrierkatalysatoren, die als Aktivmetall Ruthenium enthalten. Die katalytische Hydrierung wird vorzugsweise bei einem Wasserstoffpartialdruck von 5 bis 300 bar und einer Temperatur von 80°C bis 180°C, vorzugsweise 90°C bis 150°C durchgeführt. Der Hydrierkatalysator kann als Suspension oder als Festbett eingesetzt werden, wobei eine Rieselbetthydrierung mit einem Festbettkatalysator bevorzugt ist. Durch die Hydrierung lassen sich Probleme durch eine Zersetzung von Wasserstoffperoxid in einer destillativen Abtrennung von 1,2-Propandiol vermeiden und die in Schritt a) gebildeten Nebenprodukte 1-Hydroperoxy-2-propanol, 2-Hydroperoxy-1-propanol und Hydroxyaceton zu 1,2-Propandiol reduzieren und so die Ausbeute an 1,2-Propandiol verbessern.

Vorzugsweise wird in Schritt d) des erfindungsgemäßen Verfahrens die wässrige Phase P1 durch Nanofiltration in ein an Heteropolywolframat angereichertes Retentat und ein an Heteropolywolframat abgereichertes Permeat getrennt, das Retentat in die Umsetzung von Schritt a) zurückgeführt und aus dem Permeat 1,2-Propandiol abgetrennt. Für die Nanofiltration in Schritt d) wird eine Nanofiltrationsmembran eingesetzt, die Peroxowolframat und Heteropolyperoxowolframat im Retentat zurückhält und Wasser und 1,2-Propandiol mit dem Permeat passieren lässt. Die Nanofiltration wird dabei so geführt, dass im Retentat die Löslichkeitsgrenze des Heteropolywolframats nicht überschritten wird. Bei einer kontinuierlichen Umsetzung in Schritt a) wird vorzugsweise mit dem Retentat so viel Wasser in Schritt a) zurückgeführt, dass sich in der wässrigen Phase P1 eine Konzentration an 1,2-Propandiol im Bereich von 10 bis 30 Gew.-% einstellt. Durch eine entsprechende Rückführung von Wasser lässt sich einerseits die Bildung von Dipropylenglykol und Tripropylenglykol in Schritt a) vermeiden und andererseits die Menge an Wasser gering halten, die destillativ von 1,2-Propandiol abgetrennt werden muss. Für die Nanofiltration können Membranen auf Basis der Polymere Polyamid, Polyethersulfon, Polysulfon und Polyamidimid eingesetzt werden. Geeignete Nanofiltrationsmembranen sind kommerziell erhältlich, beispielsweise von GE Water & Process Technologies unter der Bezeichnung DK Series, von Dow Water & Process Solutions unter der Bezeichnung DOW FILMTEC™ NF, von Hydranautics unter den Bezeichnungen ESNA, ESP und SWC, von Toray Industries unter den Bezeichnungen TM700 und TM800, von SolSep unter der Bezeichnung NF 010206W, sowie von AMS Technologies unter den Bezeichnungen NanoPro™ A, NanoPro™ S und NanoPro™ B.

Zusätzlich zur Nanofiltration oder alternativ dazu kann aus der wässrigen Phase P1 Wolframat und Heteropolywolframat durch Adsorption an einem Trägermaterial entfernt werden. Vorzugsweise wird für eine solche Adsorption eines der in WO 2009/133053 auf Seite 7, Zeile 1 bis Seite 8, Zeile 29 beschriebenen kationisierten anorganischen Trägermaterialien verwendet. Die Adsorption an dem Trägermaterial und die Rückgewinnung von an dem Trägermaterial adsorbiertem Wolframat und Heteropolywolframat wird vorzugsweise mit den in WO 2009/133053 und WO 2013/110419 beschriebenen Verfahren durchgeführt. Wenn die Adsorption zusätzlich zu einer Nanofiltration in Schritt d) eingesetzt wird, wird die Adsorption vorzugsweise nach der Nanofiltration durchgeführt, um den Bedarf an Trägermaterial niedrig zu halten.

Nanofiltration und Adsorption an einem Trägermaterial werden vorzugsweise vor der oben beschriebenen Hydrierung durchgeführt, um eine Deaktivierung des Hydrierkatalysators durch Wolframat oder Heteropolywolframat zu vermeiden.

In einer bevorzugten Ausführungsform des erfindungsgemäßen Verfahrens wird in Schritt d) die in Schritt b) abgetrennte wässrige Phase P1 mit flüssigem Propen in Kontakt gebracht unter Erhalt einer wässrigen Phase P3 und einer organischen Phase P4, die organische Phase P4 wird in die Umsetzung von Schritt a) zurückgeführt und aus der wässrigen Phase P3 wird 1,2-Propandiol abgetrennt. Vorzugsweise wird bei dieser Ausführungsform keine Abtrennung von Wolfram aus der wässrigen Phase P1 durchgeführt und Wolfram wird aus der wässrigen Phase P3 abgetrennt, vorzugsweise wie zuvor für die wässrige Phase P1 beschrieben durch Nanofiltration und/oder Adsorption. Das in Kontakt bringen der wässrigen Phase P1 mit Propen erfolgt vorzugsweise in einem zusätzlichen durchmischten Reaktor mit einer Verweilzeit im Reaktor, die zu einen Umsatz des in der wässrigen Phase enthaltenen Wasserstoffperoxids von mindestens 50 %, vorzugsweise mindestens 80 %, führt. Das in Kontakt bringen in dem zusätzlichen Reaktor erfolgt vorzugsweise bei einer Temperatur von 60 bis 100 °C und einem Druck, der über dem Sättigungsdampfdruck von Propen bei der gewählten Temperatur liegt. Vorzugsweise erfolgt das in Kontakt bringen der wässrigen Phase P1 mit Propen in einem kontinuierlich betriebenen Reaktor, besonders bevorzugt in einem Schlaufenreaktor. Durch einen zusätzlichen kontinuierlich betriebenen Reaktor kann ein hoher Umsatz von Wasserstoffperoxid mit einem insgesamt geringeren Reaktorvolumen erreicht werden.

Vorzugsweise wird bei dieser Ausführungsform Propen dem Verfahren nur in Schritt d) zugeführt und gelangt mit der organischen Phase P4 in die Umsetzung von Schritt a). Wenn in dem Verfahren ein Phasentransferkatalysator eingesetzt wird, der in Gegenwart von Wasserstoffperoxid mehr als die Hälfte des Wolframs von der wässrigen Phase in flüssiges Propen transferiert, kann das in Kontakt bringen der wässrigen Phase P1 mit flüssigem Propen auch in einer Gegenstromextraktion, vorzugsweise einer Gegenstromextraktionskolonne erfolgen und mit der organischen Phase P4 wird dann in der wässrigen Phase P1 enthaltenes Wolfram in Schritt a) zurückgeführt.

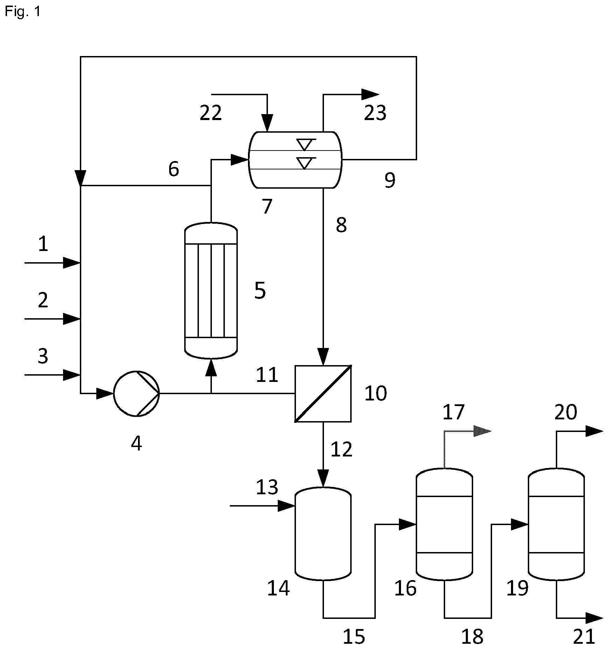

Fig. 1 zeigt eine Ausführungsform des erfindungsgemäßen Verfahrens mit einer kontinuierlichen Umsetzung in Schritt a) in einem Schlaufenreaktor und einer Nanofiltration der wässrigen Phase in Schritt d). Propen (1), Wasserstoffperoxid (2) und Katalysatormischung (3) werden einer Reaktionsschlaufe zugeführt, in der mit einer Umwälzpumpe (4) durch einen gekühlten Rohrbündelreaktor (5) die flüssige zweiphasige Mischung (6), umfassend eine wässrige Phase mit einem pH von maximal 6 und eine organische Phase, im Kreis geführt wird. Der Reaktionsschlaufe wird ein Teil der zweiphasigen Mischung (6) entnommen, der der zugeführten Menge an Propen (1), Wasserstoffperoxid (2) und Katalysatormischung (3) entspricht, und dieser Teil wird in einem Phasentrennbehälter (7) in eine wässrige Phase P1 (8) und eine organische Phase P2 (9) getrennt. Die organische Phase P2 (9) wird in die Reaktionsschlaufe zurückgeführt. Die wässrige Phase P1 (8) wird durch Nanofiltration (10) getrennt in ein an Heteropolywolframat angereichertes Retentat (11) und ein an Heteropolywolframat abgereichertes Permeat (12). Das Retentat (11) wird in die Reaktionsschlaufe zurückgeführt. Das Permeat (12) wird mit Wasserstoff (13) in einer katalytischen Hydrierung (14) umgesetzt zu einem hydrierten Permeat (15). Die Hydrierung reduziert nicht umgesetztes Wasserstoffperoxid zu Wasser und das Nebenprodukt Hydroxyaceton zu 1,2-Propandiol. Aus dem hydrierten Permeat (15) wird in einer ersten Destillation (16) Wasser (17) abdestilliert und aus dem Sumpfprodukt (18) der ersten Destillation wird in einer zweiten Destillation (19) 1,2-Propandiol (20) abdestilliert. Im Sumpf der zweiten Destillation fallen wasserlösliche Hochsieder (21) an, zum Beispiel Dipropylenglykol. Dem Gasraum des Phasentrennbehälters (7) wird Inertgas (22) zugeführt und ein sauerstoffhaltiger Gasstrom (23) entnommen, um durch Zersetzung von Wasserstoffperoxid gebildeten Sauerstoff auszuschleusen und die Bildung einer zündfähigen Gasphase im Gasraum zu vermeiden.

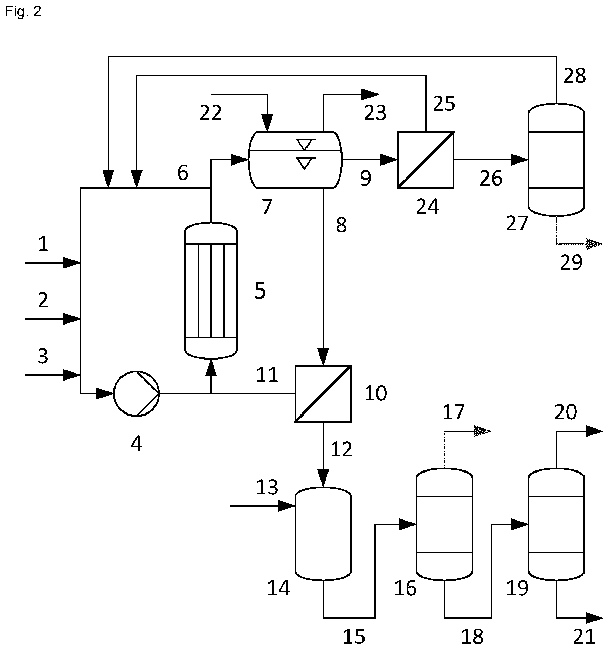

Fig. 2 zeigt eine Ausführungsform des erfindungsgemäßen Verfahrens, die zusätzlich eine Nanofiltration der organischen Phase mit anschließender Destillation in Schritt c) umfasst. Die organische Phase P2 (9) wird durch Nanofiltration (24) getrennt in ein an Heteropolywolframat angereichertes Retentat (25) und ein an Heteropolywolframat abgereichertes Permeat (26). Das Retentat (25) wird in die Umsetzung von Schritt a) zurückgeführt. Das Permeat (26) wird einer dritten Destillation (27) zugeführt, in der als Kopfprodukt ein Strom S1 (28) erhalten wird, der nicht umgesetztes Propen und als Zwischenprodukt gebildetes Propenoxid umfasst. Strom S1 (28) wird in die Reaktionsschlaufe zurückgeführt. Mit dem Sumpfprodukt (29) der dritten Destillation werden wasserunlösliche Hochsieder ausgeschleust, zum Beispiel Abbauprodukte des Phasentransferkatalysators.

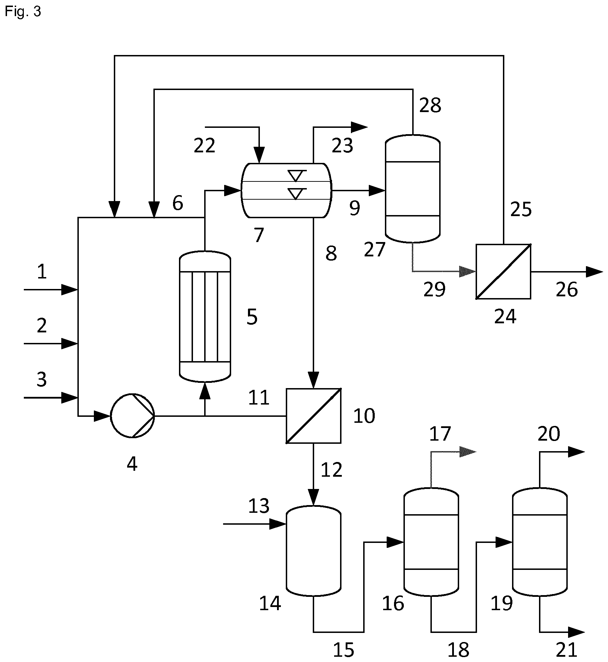

Fig. 3 zeigt eine Ausführungsform, bei der gegenüber dem Verfahren von Fig. 2 in Schritt c) die Reihenfolge von Nanofiltration und Destillation vertauscht ist. In dieser Ausführungsform wird die organische Phase P2 (9) der dritten Destillation (27) zugeführt. Der als Kopfprodukt der Destillation erhaltene Strom S1 (28), der nicht umgesetztes Propen und als Zwischenprodukt gebildetes Propenoxid umfasst, wird in die Reaktionsschlaufe zurückgeführt. Das Sumpfprodukt (29) der dritten Destillation wird durch Nanofiltration (24) getrennt in ein an Heteropolywolframat angereichertes Retentat (25) und ein an Heteropolywolframat abgereichertes Permeat (26). Das Retentat (25) wird in die Umsetzung von Schritt a) zurückgeführt.

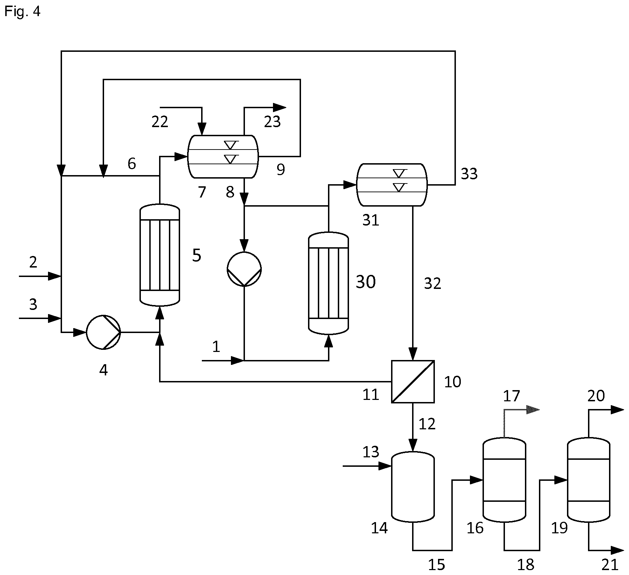

Fig. 4 zeigt eine Ausführungsform, bei der in Schritt d) die wässrige Phase P1 noch einmal in einem Reaktor mit Propen in Kontakt gebracht wird und 1,2-Propandiol aus der dabei resultierenden wässrigen Phase P3 abgetrennt wird. In dieser Ausführungsform wird die wässrige Phase P1 (8) einer zweiten Reaktionsschlaufe zugeführt, der das eingesetzte Propen (1) flüssig zugeführt wird und in der die resultierende flüssige zweiphasige Mischung mit einer Umwälzpumpe durch einen zusätzlichen Reaktor (30) im Kreis geführt wird. Der Reaktionsschlaufe wird ein Teil der zweiphasigen Mischung entnommen, der der zugeführten Menge an Propen (1) und wässriger Phase P1 (8) entspricht, und dieser Teil wird in einem Phasentrennbehälter (31) in eine wässrige Phase P3 (32) und eine organische Phase P4 (33) getrennt. Die organische Phase P4 (33) wird in die Reaktionsschlaufe der Umsetzung von Schritt a) zurückgeführt. Aus der wässrigen Phase P3 (32) wird durch Nanofiltration (10), katalytische Hydrierung (14), erste Destillation (16) und zweite Destillation (19) 1,2 Propandiol (20) abgetrennt, wie zu Fig. 1 für die wässrige Phase P1 beschrieben. Das Retentat (11) wird in die erste Reaktionsschlaufe zurückgeführt.

### Liste der Bezugszeichen in den Figuren:

- 1: Propen
- 2: Wasserstoffperoxid
- 3: Katalysatormischung
- 4: Umwälzpumpe
- 5: gekühlter Rohrbündelreaktor
- 6: zweiphasige Mischung
- 7: Phasentrennbehälter
- 8: wässrige Phase P1
- 9: organische Phase P2
- 10: Nanofiltration von wässriger Phase
- 11: Retentat der Nanofiltration 10
- 12: Permeat der Nanofiltration 10
- 13: Wasserstoff
- 14: katalytische Hydrierung
- 15: hydriertes Permeat 12
- 16: erste Destillation
- 17: Wasser
- 18: Sumpfprodukt der ersten Destillation
- 19: zweite Destillation
- 20: 1,2-Propandiol
- 21: Hochsieder
- 22: Inertgas
- 23: sauerstoffhaltiger Gasstroms
- 24: Nanofiltration von organischer Phase
- 25: Retentat der Nanofiltration 24
- 26: Permeat der Nanofiltration 24
- 27: dritte Destillation
- 28: Propen und Propenoxid enthaltender Strom
- 29: Sumpfprodukt der dritten Destillation
- 30: Zusätzlicher Reaktor
- 31: Phasentrennbehälter
- 32: wässrige Phase P3
- 33: organische Phase P4

### Beispiele

### Beispiel 1, Herstellung von epoxidiertem Sojafettsäuremethylester

In einem Rührbehälter mit 2,5l Volumen wurden 750 g Sojafettsäuremethylester, 115 g entsalztes Wasser, 13,8 g REWOQUAT® 3099 (Bis-(2-hydroxypropyl)-dimethylammoniummethylsulfat-Pflanzenfettsäurediester), 3,6 g Natriumwolframatdihydrat und 1,2 g Phosphorsäure vorgelegt. Bei 70 °C wurden unter Rühren 540 g 28 Gew.-% wässrige Wasserstoffperoxidlösung innerhalb von 1 h unter Rühren zudosiert. Die Mischung wurde weitere 1,5 h bei 70 °C gerührt, auf 20 °C gekühlt und der epoxidierte Sojafettsäuremethylester durch Phasentrennung als leichte Phase abgetrennt.

### Beispiel 2, Herstellung von 1,2-Propandiol aus Propen und Wasserstoffperoxid mit epoxidiertem Sojafettsäuremethylester als Lösungsmittel

Die Umsetzung von Propen mit Wasserstoffperoxid erfolgte bei einer Temperatur von 78 °C und einem Druck von 4,2 MPa in einem Schlaufenreaktor mit einem Volumen von 0,45 l, der mit einer Umwälzrate von 90 kg/h betrieben wurde. In den Schlaufenreaktor wurden 140 g/h Propen, 140 g/h einer 20,0 Gew.-% wässrigen Lösung von Wasserstoffperoxid, 120 g/h einer 10,0 Gew.-% Natriumwolframatdihydrat und 24,0 Gew.-% Phosphorsäure enthaltenden wässrigen Lösung, sowie 240 g/h einer Mischung aus 8,3 Gew.-% REWOQUAT® 3099 und 91,7 Gew.-% epoxidiertem Sojafettsäuremethylester aus Beispiel 1 dosiert. Dem Schlaufenreaktor wurde zweiphasige Reaktionsmischung in einer den zudosierten Mengen korrespondierenden Menge entnommen, die Mischung auf Umgebungsdruck entspannt, wobei gelöstes Propen ausgaste und anschließend die Phasen getrennt. Nach 5 h Betrieb wurden innerhalb von 30 min 301 g Reaktionsmischung entnommen und nach der Phasentrennung 138 g wässrige Phase und 163 g organische Phase erhalten. In der organischen Phase wurde durch ¹H-NMR der Gehalt an 1,2-Propandiol bestimmt. In der wässrigen Phase wurde der Gehalt an Wasserstoffperoxid durch cerimetrische Titration bestimmt. In einer Probe der wässrigen Phase wurde das Wasserstoffperoxid durch Zugabe von Natrumsulfit reduziert und anschließend die Gehalte an 1,2-Propandiol, Hydroxyaceton, Hydroxyaceton-Bisulfitaddukt, Acetaldehyd-Bisulfitaddukt, Ameisensäure und Essigsäure durch ¹H-NMR und ¹³C-NMR mit Maleinsäure als externem Standard bestimmt.

Die wässrige Phase enthielt 60 mmol/h Wasserstoffperoxid, woraus sich ein Umsatz an Wasserstoffperoxid von 93 % ergibt. Mit der organischen Phase wurden 26 mmol/h 1,2-Propandiol (3 %) erhalten, mit der wässrigen Phase 377 mmol/h (46 %) 1,2 Propandiol, 5 mmol/h Hydroxyaceton (0,6 %), 5 mmol/h Acetaldehyd, 2 mmol/h Essigsäure und 4 mmol/h Ameisensäure (die Angaben in Klammern sind auf eingesetztes Wasserstoffperoxid bezogene Ausbeuten).

### Beispiel 3, Herstellung von 1,2-Propandiol aus Propen und Wasserstoffperoxid mit epoxidiertem Sojafettsäuremethylester als Lösungsmittel

Beispiel 2 wurde wiederholt, wobei in den Schlaufenreaktor 60 g/h Propen, 140 g/h einer 25,2 Gew.-% wässrigen Lösung von Wasserstoffperoxid, 220 g/h einer 1,7 Gew.-% Natriumwolframatdihydrat und 4,0 Gew.-% Phosphorsäure enthaltenden wässrige Lösung, sowie 160 g/h einer Mischung aus 12,3 Gew.-% REWOQUAT® 3099 und 87,7 Gew.-% epoxidiertem Sojafettsäuremethylester dosiert wurden. Nach 5 h Betrieb wurden innerhalb von 30 min 292 g Reaktionsmischung entnommen und nach der Phasentrennung 204 g wässrige Phase und 88 g organische Phase erhalten.

Die wässrige Phase enthielt 534 mmol/h Wasserstoffperoxid, woraus sich ein Umsatz an Wasserstoffperoxid von 49 % ergibt. Mit der organischen Phase wurden 14 mmol/h 1,2-Propandiol (1 %) erhalten, mit der wässrigen Phase 263 mmol/h (25 %) 1,2-Propandiol, 5 mmol/h Hydroxyaceton (0,4 %), 5 mmol/h Acetaldehyd, 2 mmol/h Essigsäure und 4 mmol/h Ameisensäure.

### Beispiel 4, Herstellung von 1,2-Propandiol aus Propen und Wasserstoffperoxid mit epoxidiertem Sojafettsäuremethylester als Lösungsmittel

Beispiel 2 wurde wiederholt, wobei in den Schlaufenreaktor 120 g/h Propen, 140 g/h einer 25,1 Gew.-% wässrigen Lösung von Wasserstoffperoxid, 120 g/h einer 10,1 Gew.-% Natriumwolframatdihydrat und 24,0 Gew.-% Phosphorsäure enthaltenden wässrigen Lösung, sowie 160 g/h einer Mischung aus 8,3 Gew.-% REWOQUAT® 3099 und 91,7 Gew.-% epoxidiertem Sojafettsäuremethylester dosiert wurden. Nach 5 h Betrieb wurden innerhalb von 30 min 305 g Reaktionsmischung entnommen und nach der Phasentrennung 134 g wässrige Phase und 172 g organische Phase erhalten.

Die wässrige Phase enthielt 74 mmol/h Wasserstoffperoxid, woraus sich ein Umsatz an Wasserstoffperoxid von 93 % ergibt. Mit der organischen Phase wurden 27 mmol/h 1,2-Propandiol (3 %) erhalten, mit der wässrigen Phase 457 mmol/h (44 %) 1,2 Propandiol, 8 mmol/h Hydroxyaceton (0,8 %), 8 mmol/h Acetaldehyd, 2 mmol/h Essigsäure und 5 mmol/h Ameisensäure.

### Beispiele 5, Herstellung von 1,2-Propandiol aus Propen und Wasserstoffperoxid mit Rückführung der organischen Phase

Beispiel 2 wurde wiederholt, wobei jedoch an Stelle von epoxidiertem Sojafettsäuremethylester ein Teil der vereinigten organischen Phasen aus den Beispielen 2-4 verwendet wurde, wobei 30 g/h Propen, 93 g/h einer 29,9 Gew.-% wässrigen Lösung von Wasserstoffperoxid, 40 g/h einer 3,4 Gew.-% Natriumwolframatdihydrat und 6,9 Gew.-% Phosphorsäure enthaltenden wässrigen Lösung, sowie 160 g/h einer Mischung aus 1,5 Gew.-% REWOQUAT® 3099 und 98,5 Gew.-% vereinigte organische Phasen aus den Beispielen 2-4 dosiert wurden. Nach 5 h Betrieb wurden innerhalb von 180 min 919 g Reaktionsmischung entnommen und nach der Phasentrennung 421 g wässrige Phase und 498 g organische Phase erhalten.

Die wässrige Phase enthielt 59 mmol/h Wasserstoffperoxid, woraus sich ein Umsatz an Wasserstoffperoxid von 93 % ergibt. Mit der organischen Phase wurden 13 mmol/h 1,2-Propandiol (2 %) erhalten, mit der wässrigen Phase 290 mmol/h (35 %) 1,2 Propandiol, 16 mmol/h Hydroxyaceton (2 %), 7 mmol/h Acetaldehyd, 6 mmol/h Essigsäure und 9 mmol/h Ameisensäure.

### Beispiele 6, Herstellung von 1,2-Propandiol aus Propen und Wasserstoffperoxid mit Rückführung der organischen Phase

Beispiel 5 wurde wiederholt, wobei eine 30,4 Gew.-% wässrige Lösung von Wasserstoffperoxid verwendet wurde. Nach 5 h Betrieb wurden innerhalb von 210 min 1095 g Reaktionsmischung entnommen und nach der Phasentrennung wurden 488 g wässrige Phase und 607 g organische Phase erhalten.

Die wässrige Phase enthielt 62 mmol/h Wasserstoffperoxid, woraus sich ein Umsatz an Wasserstoffperoxid von 93 % ergibt. Mit der organischen Phase wurden 14 mmol/h 1,2-Propandiol (2 %) erhalten, mit der wässrigen Phase 288 mmol/h (35 %) 1,2 Propandiol, 17 mmol/h Hydroxyaceton (2 %), 8 mmol/h Acetaldehyd, 6 mmol/h Essigsäure und 9 mmol/h Ameisensäure.

### Beispiel 7, Abtrennung von Wolframat aus der wässrigen und der organischen Phase der Reaktionsmischung der Epoxidierung

Für die vereinigten wässrigen Phasen der Beispiel 5 und 6 und die vereinigten organischen Phasen der Beispiele 5 und 6 wurde jeweils die Abtrennung von Wolframat durch Nanofiltration untersucht.

Die Nanofiltration erfolgte als Dead-End-Filtration in einer gerührten Filtrationszelle (METcell) der Firma Evonik MET. Die Abtrennung von Wolframat aus der wässrigen Phase erfolgte mit der Membran GE DK von GE Water & Process Technologies oder der Membran NanoPro™ B-4022 von AMS Technologies. Die Abtrennung von Wolframat aus der organischen Phase erfolgte mit der Membran PuraMem® S 600 von Evonik MET oder der Membran ONF-2 von GMT Membrantechnik. Vor der Bestimmung von Rückhaltevermögen und Permeabilität wurden die Membranen für die Filtration von wässriger Phase mit Wasser und wässriger Phase und die Membranen für die Filtration von organischer Phase mit organischer Phase bei einer Rührerdrehzahl von 500 min⁻¹ unter den in Tabelle 1 angeführten Bedingungen konditioniert.

**Tabelle 1**

| Membran | Filtriertes Medium | Druck in MPa | Temperatur in °C | Filtrationsdauer in min |
|---|---|---|---|---|
| GE DK | Wasser | 2,0 | 20 | 23 |
| | wässrige Phase | 3,0 | 20 | 137 |
| NanoPro™ B-4022 | Wasser | 2,0 | 20 | 40 |
| | wässrige Phase | 3,0 | 20 | 160 |
| PuraMem® S 600 | organische Phase | 2,0 | 20 | 29 |
| | | 3,0 | 20 | 114 |
| ONF-2 | organische Phase | 3,9 | 83 | 315 |

Die Filtrationsbedingungen zur Bestimmung von Rückhaltevermögen und Permeabilität sind in den Tabellen 2-5 angeführt, die Filtrationsversuche erfolgten bei einer Rührerdrehzahl von 500 min⁻¹. Die Konzentrationen an Wolfram und Stickstoff in der eingesetzten Phase (Feed), im Retentat und im Permeat und das daraus berechnete Rückhaltevermögen für Wolframat und Phasentransferkatalysator sind in den Tabellen 6 und 7 angeführt. Das Rückhaltevermögen wurde berechnet als 1 - (Konzentration Permeat)/(Konzentration Retentat) zu dem jeweils angegebenen Zeitpunkt.

**Tabelle 2**

| Filtration von 182 g wässriger Phase mit Membran GE DK | | | | |
|---|---|---|---|---|
| Filtrationsdauer in min | Druck in MPa | Temperatur in °C | Kumulierte Masse des Permeats in g | Permeabilität in kg m⁻²h⁻¹bar⁻¹ |
| 0 | 3,0 | 20 | 0 | |
| 63 | 3,0 | 20 | 18,0 | 0,11 |
| 129 | 3,0 | 20 | 36,1 | 0,11 |
| 205 | 3,0 | 20 | 54,4 | 0,10 |
| 285 | 3,0 | 20 | 72,5 | 0,10 |
| 372 | 3,0 | 20 | 89,3 | 0,09 |

**Tabelle 3**

| Filtration von 180 g wässriger Phase mit Membran NanoPro™ B-4022 | | | | |
|---|---|---|---|---|
| Filtrationsdauer in min | Druck in MPa | Temperatur in °C | Kumulierte Masse des Permeats in g | Permeabilität in kg m⁻²h⁻¹bar⁻¹ |
| 0 | 4,5 | 20 | 0 | |
| 73 | 4,5 | 20 | 15,9 | 0,06 |
| 169 | 4,5 | 20 | 29,7 | 0,047 |
| 298 | 4,5 | 20 | 45,6 | 0,040 |
| 410 | 4,5 | 20 | 57,7 | 0,037 |

**Tabelle 4**

| Filtration von 197 g organischer Phase mit Membran PuraMem® S 600 | | | | |
|---|---|---|---|---|
| Filtrationsdauer in min | Druck in MPa | Temperatur in °C | Kumulierte Masse des Permeats in g | Permeabilität in kg m⁻²h⁻¹bar⁻¹ |
| 0 | 4,0 | 21 | 0 | |
| 385 | 4,0 | 81 | 22,1 | 0,017 |
| 745 | 4,0 | 81 | 40,4 | 0,016 |
| 1350 | 4,0 | 82 | 60,0 | 0,013 |
| 1985 | 3,9 | 80 | 75,9 | 0,011 |
| 2780 | 3,9 | 81 | 88,6 | 0,009 |

**Tabelle 5**

| Filtration von 209 g organischer Phase mit Membran ONF-2 | | | | |
|---|---|---|---|---|
| Filtrationsdauer in min | Druck in MPa | Temperatur in °C | Kumulierte Masse des Permeats in g | Permeabilität in kg m⁻²h⁻¹bar⁻¹ |
| 0 | 4,0 | 21 | 0 | |
| 930 | 4,0 | 82 | 22,8 | 0,007 |
| 1875 | 4,0 | 83 | 40,0 | 0,006 |
| 2940 | 3,9 | 82 | 57,5 | 0,005 |

**Tabelle 6**

| Nanofiltration von wässriger Phase | | | | | |
|---|---|---|---|---|---|
| Membran | Analysierte Probe | Konzentration Wolfram in ppm | Konzentration Stickstoff in ppm | Rückhalt Wolframat in % | Rückhalt PTC in % |
| GE DK | Feed | 66 | 84 | | |
| | Retentat nach 372 min | 100 | 155 | | |
| | Permeat nach 372 min | n.b. | 18 | n.b. | 84 |
| NanoPro™ B-4022 | Feed | 66 | 84 | | |
| | Retentat nach 410 min | 70 | 100 | | |
| | Permeat nach 410 min | 22 | 25 | 69 | 75 |

| | | | | | |
|---|---|---|---|---|---|
| n.b.: nicht bestimmt | | | | | |

**Tabelle 7**

| Nanofiltration von organischer Phase | | | | | |
|---|---|---|---|---|---|
| Membran | Analysierte Probe | Konzentration Wolfram in Gew.-% | Konzentration Stickstoff in ppm | Rückhalt Wolframat in % | Rückhalt PTC in % |
| PuraMem® S 600 | Feed | 2,1 | 1800 | | |
| | Retentat nach 46 min | 3,8 | 2950 | | |
| | Permeat nach 46 min | 0,4 | 450 | 89 | 85 |
| ONF-2 | Feed | 2,1 | 1800 | | |
| | Retentat nach 49 min | 2,7 | 1850 | | |
| | Permeat nach 49 min | 0,5 | 18 | 81 | 99 |

### Beispiel 8, Herstellung von 1,2-Propandiol aus Propen und Wasserstoffperoxid mit einer C10-Aromatenmischung als Lösungsmittel

Die Umsetzung von Propen mit Wasserstoffperoxid erfolgte bei einer Temperatur von 78 °C und einem Druck von 4,2 MPa in einem Schlaufenreaktor mit einem Volumen von 0,5 l, der mit einer Umwälzrate von 90 kg/h betrieben wurde. In den Schlaufenreaktor wurden 90 g/h Propen, 140 g/h einer 20,1 Gew.-% wässrigen Lösung von Wasserstoffperoxid, 120 g/h einer 10,1 Gew.-% Natriumwolframatdihydrat, 24,0 Gew.-% Phosphorsäure und 1,5 Gew.-% Wasserstoffperoxid enthaltenden wässrigen Lösung, deren pH-Wert mit festem Natriumhydroxid auf 1,5 eingestellt wurde, sowie 240 g/h einer Mischung aus 5,7 Gew.-% Trioctylamin und 94,3 Gew.-% Hydrosol A 200 ND (naphthalinarme C10-Aromatenmischung, DHC Solvent Chemie) dosiert. Dem Schlaufenreaktor wurde zweiphasige Reaktionsmischung in einer den zudosierten Mengen korrespondierenden Menge entnommen, in einem ersten Phasentrennbehälter bei 1,6 MPa 262 g/h wässrige Phase von organischer Phase und Gasphase abgetrennt und in einem zweiten Phasentrennbehälter bei dem gleichen Druck 301 g/h organische Phase von der Gasphase getrennt. In den zweiten Phasentrennbehälter wurden 50 Nl/h Stickstoff dosiert, über ein Druckhalteventil Gasphase entnommen und in dieser Gasphase der Gehalt an Sauerstoff mit einem paramagnetischen Sauerstoffsensor bestimmt. In der organischen Phase wurde ohne Druckreduzierung durch GC-MS der Gehalt an Propenoxid bestimmt. In der wässrigen Phase wurde der pH und der Gehalt an Wasserstoffperoxid durch cerimetrische Titration bestimmt. In einer Probe der wässrigen Phase wurde das Wasserstoffperoxid durch Zugabe von Natrumsulfit reduziert und anschließend die Gehalte an 1,2-Propandiol, Dipropylenglykol, Hydroxyaceton, Hydroxyaceton-Bisulfitaddukt, Acetaldehyd-Bisulfitaddukt, Ameisensäure und Essigsäure durch ¹H-NMR und ¹³C-NMR mit Maleinsäure als externem Standard bestimmt.

Mit der Gasphase wurde 1 mmol/h Sauerstoff erhalten. Die wässrige Phase hatte einen pH von 2,0 und enthielt 223 mmol/h Wasserstoffperoxid, woraus sich ein Umsatz an Wasserstoffperoxid von 75 % ergibt. Mit der organischen Phase wurden 27 mmol/h Propenoxid (3 %) erhalten, mit der wässrigen Phase 310 mmol/h (35 %) 1,2-Propandiol, 16 mmol/h (2 %) Dipropylenglykol, 13 mmol/h Hydroxyaceton (1,5 %), 11 mmol/h Acetaldehyd, 9 mmol/h Essigsäure und 6 mmol/h Ameisensäure (die Angaben in Klammern sind auf eingesetztes Wasserstoffperoxid bezogene Ausbeuten).

### Beispiel 9, Herstellung von 1,2-Propandiol aus Propen und Wasserstoffperoxid mit Rückführung der organischen Phase

Beispiel 8 wurde wiederholt, wobei in den Schlaufenreaktor 90 g/h Propen, 140 g/h einer 20,3 Gew.-% wässrigen Lösung von Wasserstoffperoxid, 120 g/h einer 1,5 Gew.-% Natriumwolframatdihydrat, 3,5 Gew.-% Phosphorsäure und 0,1 Gew.-% Wasserstoffperoxid enthaltenden wässrigen Lösung, deren pH Wert mit festem Natriumhydroxid auf 1,5 eingestellt wurde, sowie 240 g/h einer Mischung aus 0,6 Gew.-% Trioctylamin und 99,4 Gew.-% auf Umgebungsdruck entspannter organischer Phase aus Beispiel 8 dosiert wurden.

Mit der Gasphase wurden 8 mmol/h Sauerstoff erhalten. Die wässrige Phase hatte einen pH von 1,7 und enthielt 124 mmol/h Wasserstoffperoxid, woraus sich ein Umsatz an Wasserstoffperoxid von 85 % ergibt. Mit der organischen Phase wurden 25 mmol/h Propenoxid (3 %) erhalten, mit der wässrigen Phase 438 mmol/h (52 %) 1,2-Propandiol, 21 mmol/h (2,5 %) Dipropylenglykol, 24 mmol/h Hydroxyaceton (3 %), 11 mmol/h Acetaldehyd, 9 mmol/h Essigsäure und 12 mmol/h Ameisensäure.

### Beispiel 10, Herstellung von 1,2-Propandiol aus Propen und Wasserstoffperoxid mit Rückführung der organischen Phase

Beispiel 9 wurde wiederholt, wobei eine 20,0 Gew.-% wässrige Lösung von Wasserstoffperoxid und auf Umgebungsdruck entspannte organischer Phase aus Beispiel 9 verwendet wurden.

Mit der Gasphase wurden 24 mmol/h Sauerstoff erhalten. Die wässrige Phase hatte einen pH von 1,8 und enthielt 143 mmol/h Wasserstoffperoxid, woraus sich ein Umsatz an Wasserstoffperoxid von 83 % ergibt. Mit der organischen Phase wurden 26 mmol/h Propenoxid (3 %) erhalten, mit der wässrigen Phase 394 mmol/h (48 %) 1,2-Propandiol, 19 mmol/h (2,3 %) Dipropylenglykol, 21 mmol/h Hydroxyaceton (2,5 %), 12 mmol/h Acetaldehyd, 10 mmol/h Essigsäure und 6 mmol/h Ameisensäure.

### Beispiel 11, Herstellung von 1,2-Propandiol aus Propen und Wasserstoffperoxid mit einer C10-Aromatenmischung als Lösungsmittel mit Abtrennung von Wolframat aus der wässrigen und der organischen Phase

Beispiel 10 wurde wiederholt, wobei in den Schlaufenreaktor 120 g/h Propen, 140 g/h einer 20,4 Gew.-% wässrigen Lösung von Wasserstoffperoxid, 120 g/h einer 16,1 Gew.-% Natriumwolframatdihydrat, 25,6 Gew.-% Phosphorsäure und 1,5 Gew. % Wasserstoffperoxid enthaltenden wässrigen Lösung, deren pH Wert mit festem Natriumhydroxid auf 1,5 eingestellt wurde, sowie 320 g/h einer Mischung aus 2,0 Gew.-% Trioctylamin und 98,0 Gew.-% Hydrosol A 200 ND (naphthalinarme C10 Aromatenmischung, DHC Solvent Chemie) dosiert wurden.

Mit der Gasphase wurden 0,1 mmol/h Sauerstoff erhalten. Die wässrige Phase hatte einen pH von 2,0 und enthielt 58 mmol/h Wasserstoffperoxid, woraus sich ein Umsatz an Wasserstoffperoxid von 94 % ergibt. Mit der organischen Phase wurden 17 mmol/h Propenoxid (2 %) erhalten, mit der wässrigen Phase 420 mmol/h (50 %) 1,2 Propandiol, 20 mmol/h (2,4 %) Dipropylenglykol, 12 mmol/h Hydroxyaceton (1 %), 10 mmol/h Acetaldehyd, 6 mmol/h Essigsäure und 4 mmol/h Ameisensäure.

Nach dem Entspannen der Rektionsmischung und der Phasentrennung wurden für die erhaltene wässrige Phase und organische Phase separat die Abtrennung von Wolframat durch Nanofiltration untersucht. Die Nanofiltration erfolgte als Dead-End-Filtration in einer gerührten Filtrationszelle (METcell) der Firma Evonik MET. Die Abtrennung von Wolframat aus der wässrigen Phase erfolgte mit der Membran GE DK von GE Water & Process Technologies. Die Abtrennung von Wolframat aus der organischen Phase erfolgte mit der Membran ONF-2 von GMT Membrantechnik. Vor der Bestimmung von Rückhaltevermögen und Permeabilität wurden die Membranen für die Filtration von wässriger Phase mit Wasser und wässriger Phase und die Membranen für die Filtration von organischer Phase mit organischer Phase bei einer Rührerdrehzahl von 500 min⁻¹ unter den in Tabelle 8 angeführten Bedingungen konditioniert.

**Tabelle 8**

| Membran | Filtriertes Medium | Druck in MPa | Temperatur in °C | Filtrationsdauer in min |
|---|---|---|---|---|
| GE DK | Wasser | 3,0 | 20 | 17 |
| | wässrige Phase | 3,0 | 20 | 438 |
| ONF-2 | organische Phase | 3,0 | 20 | 34 |

Die Filtrationsbedingungen zur Bestimmung von Rückhaltevermögen und Permeabilität sind in den Tabellen 9 und 10 angeführt, die Filtrationsversuche erfolgten bei einer Rührerdrehzahl von 500 min⁻¹. Die Konzentrationen an Wolfram und Stickstoff in der eingesetzten Phase (Feed), im Retentat und im Permeat und das daraus berechnete Rückhaltevermögen für Wolframat und Phasentransferkatalysator sind in den Tabellen 11 und 12 angeführt. Das Rückhaltevermögen wurde berechnet als 1 - (Konzentration Permeat)/(Konzentration Retentat) zu dem jeweils angegebenen Zeitpunkt.

**Tabelle 9**

| Filtration von 183 g wässriger Phase mit Membran GE DK | | | | |
|---|---|---|---|---|
| Filtrationsdauer in min | Druck in MPa | Temperatur in °C | Kumulierte Masse des Permeats in g | Permeabilität in kg m⁻²h⁻¹bar⁻¹ |
| 0 | 3,0 | 24 | 0 | |
| 98 | 3,0 | 24 | 18,0 | 0,071 |
| 198 | 3,0 | 24 | 36,2 | 0,071 |
| 314 | 3,0 | 24 | 54,4 | 0,067 |
| 470 | 3,0 | 24 | 72,8 | 0,062 |
| 560 | 3,0 | 24 | 90,0 | 0,062 |

**Tabelle 10**

| Filtration von 200 g organischer Phase mit Membran ONF-2 | | | | |
|---|---|---|---|---|
| Filtrationsdauer in min | Druck in MPa | Temperatur in °C | Kumulierte Masse des Permeats in g | Permeabilität in kg m⁻²h⁻¹bar⁻¹ |
| 0 | 3,0 | 25 | 0 | |
| 10 | 3,0 | 25 | 20,2 | 0,772 |
| 21 | 3,0 | 25 | 40,0 | 0,735 |
| 32 | 3,0 | 25 | 59,8 | 0,710 |
| 46 | 3,0 | 25 | 79,5 | 0,667 |
| 62 | 3,0 | 25 | 100,3 | 0,625 |

**Tabelle 11**

| Nanofiltration von wässriger Phase | | | | | |
|---|---|---|---|---|---|
| Membran | Analysierte Probe | Konzentration Wolfram in ppm | Konzentration Stickstoff in ppm | Rückhalt Wolframat in % | Rückhalt PTC in % |
| GE DK | Feed | 21000 | 51 | | |
| | Retentat nach 560 min | 40000 | 58 | | |
| | Permeat nach 560 min | 2800 | 39 | 93 | 33 |

**Tabelle 12**

| Nanofiltration von organischer Phase | | | | | |
|---|---|---|---|---|---|
| Membran | Analysierte Probe | Konzentration Wolfram in ppm | Konzentration Stickstoff in ppm | Rückhalt Wolframat in % | Rückhalt PTC in % |
| ONF-2 | Feed | 15000 | 1650 | | |
| | Retentat nach 62 min | 29000 | 3300 | | |
| | Permeat nach 62 min | 7 | 210 | 99,998 | 94 |

## Patentansprüche

1. Verfahren zur Herstellung von 1,2-Propandiol aus Propen und Wasserstoffperoxid umfassend die Schritte
a) Umsetzen von Propen (1) mit Wasserstoffperoxid (2) in Gegenwart einer Katalysatormischung (3) umfassend einen Phasentransferkatalysator und ein Heteropolywolframat, wobei die Umsetzung in einer flüssigen Mischung (6) umfassend eine wässrige Phase mit einem pH von maximal 6 und eine organische Phase durchgeführt wird,
b) Trennen der zweiphasigen Mischung (6) von Schritt a) in eine wässrige Phase P1 (8) und eine Propenoxid enthaltende organische Phase P2 (9),
c) Rückführen des in der abgetrennten organischen Phase P2 (9) enthaltenen Propenoxids in die Umsetzung von Schritt a) und
d) Abtrennen von 1,2-Propandiol (20) aus der in Schritt b) abgetrennten wässrigen Phase P1 (8).

2. Verfahren nach Anspruch 1, **dadurch gekennzeichnet, dass** in Schritt c) das in der abgetrennten organischen Phase P2 (9) enthaltene Heteropolywolframat in die Umsetzung von Schritt a) zurückgeführt wird.

3. Verfahren nach Anspruch 1 oder 2, **dadurch gekennzeichnet, dass** in Schritt a) der pH der wässrigen Phase im Bereich von 1,0 bis 3,5, vorzugsweise von 2,0 bis 3,0, gehalten wird.

4. Verfahren nach einem der Ansprüche 1 bis 3, **dadurch gekennzeichnet, dass** in Schritt d) die wässrige Phase P1 (8) durch Nanofiltration (10) getrennt wird in ein an Heteropolywolframat angereichertes Retentat (11) und ein an Heteropolywolframat abgereichertes Permeat (12), das Retentat (11) in die Umsetzung von Schritt a) zurückgeführt wird und aus dem Permeat (12) 1,2-Propandiol (20) abgetrennt wird.

5. Verfahren nach einem der Ansprüche 1 bis 4, **dadurch gekennzeichnet, dass** in Schritt a) die Umsetzung in Gegenwart von mindestens einem Lösungsmittel durchgeführt wird, das einen Siedepunkt von mehr als 100 °C und eine Wasserlöslichkeit bei 20 °C von weniger als 250 mg/kg aufweist.

6. Verfahren nach Anspruch 5, **dadurch gekennzeichnet, dass** das Lösungsmittel einen epoxidierten Fettsäuremethylester umfasst.

7. Verfahren nach Anspruch 5, **dadurch gekennzeichnet, dass** das Lösungsmittel einen alkylierten aromatischen Kohlenwasserstoff mit 8 bis 12 Kohlenstoffatomen umfasst.

8. Verfahren nach einem der Ansprüche 1 bis 7, **dadurch gekennzeichnet, dass** die in Schritt b) abgetrennte organische Phase P2 (9) vollständig oder zum Teil in die Umsetzung von Schritt a) zurückgeführt wird.

9. Verfahren nach einem der Ansprüche 1 bis 8, **dadurch gekennzeichnet, dass** in Schritt c) die organische Phase P2 (9) ganz oder teilweise durch Nanofiltration (24) in ein an Heteropolywolframat angereichertes Retentat (25) und ein an Heteropolywolframat abgereichertes Permeat (26) getrennt wird und das Retentat (25) in die Umsetzung von Schritt a) zurückgeführt wird.

10. Verfahren nach Anspruch 9, **dadurch gekennzeichnet, dass** die organische Phase P2 (9) durch Nanofiltration (24) in das an Heteropolywolframat angereicherte Retentat (25) und das an Heteropolywolframat abgereicherte Permeat (26) getrennt wird, aus dem Permeat (26) durch Destillation (27) ein Strom S1 (28), umfassend nicht umgesetztes Propen und als Zwischenprodukt gebildetes Propenoxid, abgetrennt wird und dieser Strom S1 (28) in die Umsetzung von Schritt a) zurückgeführt wird.

11. Verfahren nach Anspruch 9, **dadurch gekennzeichnet, dass** die organische Phase P2 (9) durch Destillation (27) aufgetrennt wird in einen Strom S1 (28), umfassend nicht umgesetztes Propen und als Zwischenprodukt gebildetes Propenoxid, sowie einen an Propen und Propenoxid abgereicherten Strom S2 (29), der Strom S2 (29) durch Nanofiltration (24) in das an Heteropolywolframat angereicherte Retentat (25) und das an Heteropolywolframat abgereicherte Permeat (26) getrennt wird und der Strom S1 (28) in die Umsetzung von Schritt a) zurückgeführt wird.

12. Verfahren nach einem der Ansprüche 1 bis 11, **dadurch gekennzeichnet, dass** in Schritt d) vor der Abtrennung von 1,2-Propandiol Peroxide durch eine katalytische Hydrierung (14) entfernt werden.

13. Verfahren nach einem der Ansprüche 1 bis 12, **dadurch gekennzeichnet, dass** in Schritt d) die in Schritt b) abgetrennte wässrige Phase P1 (8) mit flüssigem Propen (1) in Kontakt gebracht wird unter Erhalt einer wässrigen Phase P3 (32) und einer organischen Phase P4 (33), die organische Phase P4 (33) in die Umsetzung von Schritt a) zurückgeführt wird und aus der wässrigen Phase P3 (34) 1,2-Propandiol abgetrennt wird.

14. Verfahren nach Anspruch 13, **dadurch gekennzeichnet, dass** Propen (1) dem Verfahren nur in Schritt d) zugeführt wird.

15. Verfahren nach einem der Ansprüche 1 bis 14, **dadurch gekennzeichnet, dass** Propen in Mischung mit Propan eingesetzt wird.

16. Verfahren nach einem der Ansprüche 1 bis 15, **dadurch gekennzeichnet, dass** Schritt a) kontinuierlich durchgeführt wird und die Konzentration an Wasserstoffperoxid in der wässrigen Phase im Bereich von 0,1 bis 5 Gew.-% liegt.

17. Verfahren nach einem der Ansprüche 1 bis 16, **dadurch gekennzeichnet, dass** Schritt a) kontinuierlich in einem Schlaufenreaktor durchgeführt wird, der unbewegliche Einbauten aufweist, und die flüssige Mischung (6) mit einer Strömungsgeschwindigkeit durch den Schlaufenreaktor geführt wird, die an den Einbauten eine turbulente Strömung erzeugt.

18. Verfahren nach einem der Ansprüche 1 bis 17, **dadurch gekennzeichnet, dass** das Heteropolywolframat ein Polywolframatophosphat ist.

19. Verfahren nach Anspruch 18, **dadurch gekennzeichnet, dass** das Polywolframatophosphat in Schritt a) in situ aus Phosphorsäure und Natriumwolframat erzeugt wird.

20. Verfahren nach Anspruch 19, **dadurch gekennzeichnet, dass** Phosphorsäure und Natriumwolframat in einem molaren Verhältnis von 1:2 bis 10:1 eingesetzt werden.

21. Verfahren nach einem der Ansprüche 1 bis 20, **dadurch gekennzeichnet, dass** der Phasentransferkatalysator mindestens ein Salz mit einem tertiären oder quaternären Ammoniumion der Struktur R¹R²R³R⁴N⁺ umfasst, worin
R¹ ein Rest Y-O(C=O)R⁵ ist, wobei Y für einen der Reste CH₂CH₂, CH(CH₃)CH₂ und CH₂CH(CH₃) steht und R⁵ ein Alkylrest oder Alkenylrest mit 11 bis 21 Kohlenstoffatomen ist,
R² Wasserstoff oder ein Alkylrest mit 1 bis 4 Kohlenstoffatomen ist und
R³ und R⁴ unabhängig voneinander R¹, ein Alkylrest mit 1 bis 4 Kohlenstoffatomen oder Y-OH sind.

22. Verfahren nach einem der Ansprüche 1 bis 21, **dadurch gekennzeichnet, dass** Schritt b) in Gegenwart einer Gasphase durchgeführt wird und durch Zufuhr von Inertgas (22) und Entnahme eines Gasstroms (23) der Sauerstoffgehalt dieser Gasphase auf weniger als 7 Vol. % gehalten wird.

## Claims

1. Process for preparing 1,2-propanediol from propene and hydrogen peroxide comprising the steps of
a) reacting propene (1) with hydrogen peroxide (2) in the presence of a catalyst mixture (3) comprising a phase transfer catalyst and a heteropolytungstate, wherein the reaction is carried out in a liquid mixture (6) comprising an aqueous phase having a pH of at most 6 and an organic phase,
b) separating the biphasic mixture (6) from step a) into an aqueous phase P1 (8) and an organic phase P2 (9) comprising propene oxide,
c) recycling the propene oxide present in the separated organic phase P2 (9) into the reaction of step a) and
d) separating 1,2-propanediol (20) from the aqueous phase P1 (8) separated in step b).

2. Process according to Claim 1, **characterized in that** the heteropolytungstate present in the organic phase P2 (9) separated in step c) is recycled into the reaction of step a).

3. Process according to Claim 1 or 2, **characterized in that** the pH of the aqueous phase in step a) is maintained in the range of 1.0 to 3.5, preferably 2.0 to 3.0.

4. Process according to any of Claims 1 to 3, **characterized in that** the aqueous phase P1 (8) is separated by nanofiltration (10) in step d) into a retentate (11) enriched in heteropolytungstate and a permeate (12) depleted in heteropolytungstate, the retentate (11) is recycled into the reaction of step a) and 1,2-propanediol (20) is separated from the permeate (12).

5. Process according to any one of Claims 1 to 4, **characterized in that** the reaction in step a) is carried out in the presence of at least one solvent having a boiling point of more than 100°C and a water solubility at 20°C of less than 250 mg/kg.

6. Process according to Claim 5, **characterized in that** the solvent comprises an epoxidized fatty acid methyl ester.

7. Process according to Claim 5, **characterized in that** the solvent comprises an alkylated aromatic hydrocarbon having 8 to 12 carbon atoms.

8. Process according to any one of Claims 1 to 7, **characterized in that** the organic phase P2 (9) separated in step b) is completely or partly recycled into the reaction of step a).

9. Process according to any one of Claims 1 to 8, **characterized in that** the organic phase P2 (9) is separated completely or partly by nanofiltration (24) in step c) into a retentate (25) enriched in heteropolytungstate and a permeate (26) depleted in heteropolytungstate and the retentate (25) is recycled into the reaction of step a).

10. Process according to Claim 9, **characterized in that** the organic phase P2 (9) is separated by nanofiltration (24) into the retentate (25) enriched in heteropolytungstate and the permeate (26) depeleted in heteropolytungstate, a stream S1 (28) comprising unreacted propene and propene oxide formed as intermediate is separated by distillation (27) from the permeate (26) and this stream S1 (28) is recycled into the reaction of step a).

11. Process according to Claim 9, **characterized in that** the organic phase P2 (9) is separated by distillation (27) into a stream S1 (28) comprising unreacted propene and propene oxide formed as intermediate, and also a stream S2 (29) depleted in propene and propene oxide, the stream S2 (29) is separated by nanofiltration (24) into the retentate (25) enriched in heteropolytungstate and the permeate (26) depleted in heteropolytungstate and the stream S1 (28) is recycled into the reaction of step a).

12. Process according to any one of Claims 1 to 11, **characterized in that** in step d), prior to the separation of 1,2-propanediol, peroxides are removed by catalytic hydrogenation (14).

13. Process according to any one of Claims 1 to 12, **characterized in that** the aqueous phase P1 (8) separated in step b) is brought into contact with liquid propene (1) in step d) to obtain an aqueous phase P3 (32) and an organic phase P4 (33), the organic phase P4 (33) is recycled into the reaction of step a) and 1,2-propanediol is separated from the aqueous phase P3 (34).

14. Process according to Claim 13, **characterized in that** propene (1) is supplied to the process only in step d) .

15. Process according to any one of Claims 1 to 14, **characterized in that** propene is used in a mixture with propane.

16. Process according to any one of Claims 1 to 15, **characterized in that** step a) is carried out continuously and the concentration of hydrogen peroxide in the aqueous phase is in the range of 0.1 to 5% by weight.

17. Process according to any one of Claims 1 to 16, **characterized in that** step a) is carried out continuously in a loop reactor having fixed internals, and the liquid mixture (6) is conducted through the loop reactor at a flow rate which generates a turbulent flow over the internals.

18. Process according to any one of Claims 1 to 17, **characterized in that** the heteropolytungstate is a polytungstophosphate.

19. Process according to Claim 18, **characterized in that** the polytungstophosphate in step a) is generated in situ from phosphoric acid and sodium tungstate.

20. Process according to Claim 19, **characterized in that** phosphoric acid and sodium tungstate are used in a molar ratio of from 1:2 to 10:1.

21. Process according to any one of Claims 1 to 20, **characterized in that** the phase transfer catalyst comprises at least one salt having a tertiary or quaternary ammonium ion of the structure R¹R²R³R⁴N⁺, where
R¹ is a Y-O(C=O)R⁵ group, where Y is one of the groups CH₂CH₂, CH(CH₃)CH₂ and CH₂CH(CH₃) and R⁵ is an alkyl group or alkenyl group having 11 to 21 carbon atoms,
R² is hydrogen or an alkyl group having 1 to 4 carbon atoms and
R³ and R⁴ are each independently R¹, an alkyl group having 1 to 4 carbon atoms or Y-OH.

22. Process according to any one of Claims 1 to 21, **characterized in that** step b) is carried out in the presence of a gas phase and the oxygen content of this gas phase is maintained at less than 7% by volume by supplying inert gas (22) and withdrawal of a gas stream (23).

## Revendications

1. Procédé de fabrication de 1,2-propanediol à partir de propène et de peroxyde d'hydrogène, comprenant les étapes suivantes :
a) la mise en réaction de propène (1) avec du peroxyde d'hydrogène (2) en présence d'un mélange catalytique (3) comprenant un catalyseur de transfert de phase et un hétéropolytungstate, la mise en réaction étant réalisée dans un mélange liquide (6) comprenant une phase aqueuse ayant un pH d'au plus 6 et une phase organique,
b) la séparation du mélange biphasé (6) de l'étape a) en une phase aqueuse P1 (8) et une phase organique contenant de l'oxyde de propène P2 (9),
c) le recyclage de l'oxyde de propène contenu dans la phase organique séparée P2 (9) dans la réaction de l'étape a), et
d) la séparation de 1,2-propanediol (20) de la phase aqueuse P1 (8) séparée à l'étape b).

2. Procédé selon la revendication 1, **caractérisé en ce qu'**à l'étape c), l'hétéropolytungstate contenu dans la phase organique P2 séparée (9) est recyclé dans la réaction de l'étape a).

3. Procédé selon la revendication 1 ou 2, **caractérisé en ce qu'**à l'étape a), le pH de la phase aqueuse est maintenu dans la plage allant de 1,0 à 3,5, de préférence de 2,0 à 3,0.

4. Procédé selon l'une quelconque des revendications 1 à 3, **caractérisé en ce qu'**à l'étape d), la phase aqueuse P1 (8) est séparée par nanofiltration (10) en un rétentat (11) enrichi en hétéropolytungstate et un perméat (12) appauvri en hétéropolytungstate, le rétentat (11) est recyclé dans la réaction de l'étape a) et le 1,2-propanediol (20) est séparé du perméat (12).

5. Procédé selon l'une quelconque des revendications 1 à 4, **caractérisé en ce qu'**à l'étape a), la réaction est réalisée en présence d'au moins un solvant, qui présente un point d'ébullition de plus de 100 °C et une solubilité dans l'eau à 20 °C de moins de 250 mg/kg.

6. Procédé selon la revendication 5, **caractérisé en ce que** le solvant comprend un ester méthylique d'acide gras époxydé.

7. Procédé selon la revendication 5, **caractérisé en ce que** le solvant comprend un hydrocarbure aromatique alkylé ayant de 8 à 12 atomes de carbone.

8. Procédé selon l'une quelconque des revendications 1 à 7, **caractérisé en ce que** la phase organique P2 (9) séparée à l'étape b) est recyclée entièrement ou partiellement dans la réaction de l'étape a).

9. Procédé selon l'une quelconque des revendications 1 à 8, **caractérisé en ce qu'**à l'étape c), la phase organique P2 (9) est séparée entièrement ou partiellement par nanofiltration (24) en un rétentat (25) enrichi en hétéropolytungstate et un perméat (26) appauvri en hétéropolytungstate, et le rétentat (25) est recyclé dans la réaction de l'étape a).

10. Procédé selon la revendication 9, **caractérisé en ce que** la phase organique P2 (9) est séparée par nanofiltration (24) en un rétentat (25) enrichi en hétéropolytungstate et un perméat (26) appauvri en hétéropolytungstate, un courant S1 (28) comprenant du propène non réagi et de l'oxyde de propène formé en tant que produit intermédiaire est séparé du perméat (26) par distillation (27), et ce courant S1 (28) est recyclé dans la réaction de l'étape a).

11. Procédé selon la revendication 9, **caractérisé en ce que** la phase organique P2 (9) est séparée par distillation (27) en un courant S1 (28), comprenant du propène non réagi et de l'oxyde de propène formé en tant que produit intermédiaire, ainsi qu'en un courant S2 (29) appauvri en propène et en oxyde de propène, le courant S2 (29) est séparé par nanofiltration (24) en le rétentat (25) enrichi en hétéropolytungstate et le perméat (26) appauvri en hétéropolytungstate, et le courant S1 (28) est recyclé dans la réaction de l'étape a).

12. Procédé selon l'une quelconque des revendications 1 à 11, **caractérisé en ce qu'**à l'étape d), des peroxydes sont éliminés par hydrogénation catalytique (14) avant la séparation du 1,2-propanediol.

13. Procédé selon l'une quelconque des revendications 1 à 12, **caractérisé en ce qu'**à l'étape d), la phase aqueuse P1 (8) séparée à l'étape b) est mise en contact avec du propène liquide (1) pour obtenir une phase aqueuse P3 (32) et une phase organique P4 (33), la phase organique P4 (33) est recyclée dans la réaction de l'étape a) et le 1,2-propanediol est séparé de la phase aqueuse P3 (34) .

14. Procédé selon la revendication 13, **caractérisé en ce que** du propène (1) n'est introduit dans le procédé qu'à l'étape d).

15. Procédé selon l'une quelconque des revendications 1 à 14, **caractérisé en ce que** le propène est utilisé en mélange avec du propane.

16. Procédé selon l'une quelconque des revendications 1 à 15, **caractérisé en ce que** l'étape a) est réalisée en continu et la concentration en peroxyde d'hydrogène dans la phase aqueuse se situe dans la plage allant de 0,1 à 5 % en poids.

17. Procédé selon l'une quelconque des revendications 1 à 16, **caractérisé en ce que** l'étape a) est réalisée en continu dans un réacteur à boucle, qui comprend des composants internes immobiles, et le mélange liquide (6) est acheminé au travers du réacteur à boucle à une vitesse d'écoulement qui génère un écoulement turbulent au niveau des composants internes.

18. Procédé selon l'une quelconque des revendications 1 à 17, **caractérisé en ce que** l'hétéropolytungstate est un polytungstophoshate.

19. Procédé selon la revendication 18, **caractérisé en ce que** le polytungstophosphate est formé in situ à l'étape a) à partir d'acide phosphorique et de tungstate de sodium.

20. Procédé selon la revendication 19, **caractérisé en ce que** l'acide phosphorique et le tungstate de sodium sont utilisés en un rapport molaire de 1:2 à 10:1.

21. Procédé selon l'une quelconque des revendications 1 à 20, **caractérisé en ce que** le catalyseur de transfert de phase comprend au moins un sel avec un ion ammonium tertiaire ou quaternaire de structure R¹R²R³R⁴N⁺, dans laquelle
R¹ est un radical Y-O(C=O)R⁵, Y représentant un des radicaux CH₂CH₂, CH(CH₃)CH₂ et CH₂CH(CH₃), et R⁵ étant un radical alkyle ou un radical alcényle ayant de 11 à 21 atomes de carbone,
R² est l'hydrogène ou un radical alkyle ayant de 1 à 4 atomes de carbone, et
R³ et R⁴ sont indépendamment l'un de l'autre R¹, un radical alkyle ayant de 1 à 4 atomes de carbone ou Y-OH.

22. Procédé selon l'une quelconque des revendications 1 à 21, **caractérisé en ce que** l'étape b) est réalisée en présence d'une phase gazeuse et, par introduction d'un gaz inerte (22) et soutirage d'un courant gazeux (23), la teneur en oxygène de cette phase gazeuse est maintenue à moins de 7 % en volume.
